(19) Europäisches Patentamt
European Patent Office
Office européen des brevets

(11) EP 4 312 851 B1

(12) **EUROPEAN PATENT SPECIFICATION**

(45) Date of publication and mention of the grant of the patent:
**09.10.2024 Bulletin 2024/41**

(21) Application number: **22712075.5**

(22) Date of filing: **22.03.2022**

(51) International Patent Classification (IPC):
***A61B 17/29*** (2006.01) ***A61B 34/00*** (2016.01)
*A61B 90/92* (2016.01) *A61B 90/00* (2016.01)

(52) Cooperative Patent Classification (CPC):
**A61B 34/70; A61B 17/29; A61B 90/92;**
A61B 2017/00115; A61B 2017/2926;
A61B 2090/065; A61B 2090/0807

(86) International application number:
**PCT/IB2022/052608**

(87) International publication number:
**WO 2022/201024 (29.09.2022 Gazette 2022/39)**

(54) **MECHANOCHROMIC PRESSURE SENSOR FOR SAFE AND EFFECTIVE TISSUE HANDLING IN MINIMALLY INVASIVE SURGERY**

MECHANOCHROMER DRUCKSENSOR ZUR SICHEREN UND EFFEKTIVEN GEWEBEHANDHABUNG IN DER MINIMALINVASIVEN CHIRURGIE

CAPTEUR DE PRESSION MÉCANOCHROMIQUE POUR LA MANIPULATION SÛRE ET EFFICACE DE TISSU EN CHIRURGIE MINI-INVASIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2021 IT 202100006983**

(43) Date of publication of application:
**07.02.2024 Bulletin 2024/06**

(73) Proprietor: **Fondazione Istituto Italiano di Tecnologia**
**16163 Genova (IT)**

(72) Inventors:
- **GIORDANO, Goffredo**
  **56125 Pisa (IT)**
- **SINIBALDI, Edoardo**
  **56025 Pontedera PI (IT)**
- **HUAN, Yu**
  **56025 Pontedera PI (IT)**
- **GAGLIARDI, Mariacristina**
  **56017 San Giuliano Terme PI (IT)**
- **CARLOTTI, Marco**
  **56125 Pisa (IT)**
- **MAZZOLAI, Barbara**
  **56025 Pontedera PI (IT)**

(74) Representative: **Papa, Elisabetta et al**
**Società Italiana Brevetti S.p.A**
**Piazza di Pietra, 39**
**00186 Roma (IT)**

(56) References cited:
**US-A1- 2003 216 732**
**US-A1- 2019 151 502**
**US-A1- 2021 041 314**
**US-A1- 2021 059 701**

- **GREGORY R. GOSSWEILER ET AL: "Mechanochemical Activation of Covalent Bonds in Polymers with Full and Repeatable Macroscopic Shape Recovery", ACS MACRO LETTERS, vol. 3, no. 3, 12 February 2014 (2014-02-12), pages 216 - 219, XP055368186, ISSN: 2161-1653, DOI: 10.1021/mz500031q**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 312 851 B1

## Description

### Technical field

The present invention relates to a mechanochromic indicator, or sensor, for a tissue or organ manipulation device used in Minimally Invasive (Robotic) Surgery (MIS/MIRS). The invention relates as well to a manipulation device, in particular a mechanical end-effector and more particularly a miniaturized grasper or retractor devised to grasp or retract a soft tissue, including the mechanochromic indicator or sensor.

### Background

Minimally Invasive Surgery (MIS) refers to any surgical procedure that is performed through tiny incisions instead of a large opening. MIS provides several advantages over the "classical" open procedures, such as less pain, minimal to no scars, less blood loss, lower rate of complications, shorter hospital stay.

Known techniques of MIS usually employ several tools, including a mechanical end-effector such as a grasper, for the manipulation of tissue and/or organ. The end-effector is generally introduced *in situ* through the lumen of a cannula and may be manually or robotically controlled. An endoscope is also generally used to provide the surgeon with an illumination and a continuous visual monitoring over the intervention area.

The main drawback of existing tools is that they fail to provide the surgeon with an appropriate qualitative and/or quantitative feedback about the forces applied to the tissue by the end-effector and, therefore, about the safe handling thereof.

In fact, in principle robotic platforms can augment surgeon's dexterity and accuracy, but the lack of proper awareness about the force that is applied during the surgical actions still remains a crucial issue.

In particular, a visual feedback that avoids the use of electronics elements in the end-effector would be preferred, because such elements require wiring connections that might entail expensive and complex structures to allow, e.g., sterilization. In addition, electronic elements entail criticalities in terms, e.g., of algorithmic compensation of cross-talk loads.

In the state of the art, several attempts have been made to meet the above need. WO2016056908A1 discloses a mechanical end-effector provided with bendable members. The flexing of the latter, i.e. their deformation under load, is used to provide a visual feedback to the operator. A further protruding visual indicator or marker is also applied on the bendable members.

This solution provides several drawbacks, since the described visual feedback is not easy discernible and strongly dependent upon the illumination conditions. Moreover, the protruding indicator may cause potential harm to the surrounding tissues or organs. In addition, the grasping pressure, and thus the slippage risk, can not be detected. US20190151502A1 discloses a medical product having a coating layer containing a mechanophore element which, under stress, undergoes a colour-changing. However, the modes and threshold of activation of such element are not disclosed.

US 2003/216732 A1 discloses a medical device for creating thermal welds in engaged tissues or fastening tissues, which is characterized by a tissue-engaging surface that carries a thermochromic material or piezochromic material in a working surface.

US 2021/041314 A1 discloses a surgical instrument comprising an end effector and a region of stress-responsive colorimetric material on the end effector. The colorimetric material has a color that changes with increases in pressure applied to tissue by the end effector and is positioned to be visible to a user or endoscopic camera when in a body cavity engaging tissue.

### Summary of the invention

The technical problem posed and solved by the present invention is to overcome one or more of the drawbacks previously mentioned with reference to the known art.

Such a problem is solved by a manipulation device for MIS procedures, in particular an end-effector, according to claim 1.

Preferred features of the present invention are the object of the dependent claims.

The invention comprises a mechanochromic indicator device, or indicator, for a mechanical end-effector used in minimally invasive (possibly robotic) surgery.

The term "mechanochromic" used in the context of the present invention refers to a material capable of changing colour when subjected to mechanical stimuli and/or a triggering force.

The expression "mechanical end-effector" indicates herein tools usually employed in minimally invasive surgery that are generally connected to a handle by means of a support shaft. Such tools may be manually or robotically operated

in order to manipulate tissues and/or organs. According to a preferred embodiment, the mechanical end-effector is, or includes, a miniaturized grasper.

The mechanical end-effector, in a working condition, exerts a force upon a soft tissue and/or organ being manipulated. When the manipulation force is equal to - or just below / close to - the damage threshold of that tissue and/or organ, the indicator provides a visual feedback to the operator, typically the surgeon.

To this aim, the mechanical end-effector may be provided with force transmission means, in particular a force transmission mechanism, acting upon the mechanochromic indicator. A triggering force, or pressure, is thus applied upon the indicator to produce a colour change thereof. The latter may be detectable by naked eye.

Accordingly, the invention makes possible to prevent any unwanted damages to tissues and/or organs during their surgical manipulation by alarming a surgeon through said colour change.

According to a preferred embodiment, the mechanical end-effector comprises a tool suitable for integration into a da Vinci surgical robotic system (or, equivalently, to a da Vinci Research Kit - dVRK - platform), functionalized with the adaptively morphing grasper disclosed in WO2019155316A1.

Herein, the terms "distal" and "proximal" are used to denote a remoter or closer position, respectively, from the tissue/organ to be manipulated.

The indicator is made of at least one polymeric matrix functionalized with mechanochromic molecules. The mechanochromic molecules may be defined as molecules that are capable of changing colour when subjected to mechanical stimuli and/or a triggering force.

The prepolymer/curing agent weight ratio of the at least one matrix and the percentage by weight of the mechanochromic molecules in the at least one matrix have been engineered in order to make the end-effector suitable for alarming a surgeon when the damage threshold of the tissue and/or organ under manipulation has been reached. The indication is provided via a naked eye-detectable colour change.

Preferably, the indicator is arranged directly onto a proximal (grasping) portion of the surgical end-effector, e.g. at a jaw member thereof.

As mentioned above, the invention provides a suitable force transmission mechanism between a grasping portion of the end-effector and the mechanochromic indicator arrangement.

As mentioned, the invention provides a colour-coded visual feedback, giving information of excessive force or pressure applied onto a tissue being manipulated. Accordingly, the invention provides an immediate and direct indication of the stress applied onto the tissue, with high visual discernibility. Preferably, the invention also allows indicating a slippage risk as associated with the pressure exerted onto the tissue.

Advantageously, the above visual feedback can be provided by a simple structure, perfectly integrated in the end-effector.

The invention may also allow overcoming the drawbacks associated with electronic-based sensing systems.

Additional features and advantages of the various aspects of the present invention will become apparent from the following detailed description of its preferred embodiments, together with the accompanying figures. Preferred embodiments are intended to explain and exemplify certain aspects of the present of invention but should not be construed as limiting its scope of protection.

**Brief description of the drawings**

The present invention and the following detailed description of preferred embodiments thereof may be better understood with reference to the following figures:

- Figures 1A and 1B relate to a manipulation device for MIS according to a first embodiment of the present invention, showing an exemplary perspective assembled view and an exemplary exploded perspective view thereof, respectively;

- Fig. 1C and 1D show each a front view of a jaw device of the manipulation device of Fig. 1A, in a respective configuration during grasping of a tissue;

- Fig. 1E shows a perspective view of the manipulation device of Fig. 1A, with an exemplary indication of relevant forces and dimensions;

- Figures 2A, 2B and 2C relate to a manipulation device for MIS according to a variant embodiment of the present invention, showing an exemplary perspective view thereof, an exemplary enlarged view of a detail of Figure 2A and an exemplary graph of a colorimetric spectroscopic data obtained from said manipulation device, respectively;

- Figure 3 exemplifies the use of a manipulation device according to an embodiment of the present invention during

an abdominal surgical intervention;

- Figure 4 shows an exemplary perspective view of a component of a manipulation device according to a variant embodiment of the present invention;

- Figures 5A and 5B relate to a manipulation device for MIS according to another embodiment of the present invention, showing an exemplary perspective view thereof and an exemplary enlarged view of a detail of Figure 5A, respectively;

- Figure 6 is a graphical representation of the mechanochromic spiropyran molecules covalently bonded to the polymeric matrix. The mechanical indentation returns the spiro C-O covalent bond break, reporting the classical SP->MC coloured reversible reaction;

- Figure 7 shows the temperature dependency of the merocyanine (MC) -> spiropyran (SP) recovery for a thin film of 2%wt. SP-doped PDMS (2.5:1);

- Figure 8 shows 1H NMR spectrum of bis-functionalized SP monomer (400 MHz, CDCl3) $\delta$ 7.69 (d, 1H), 7.60 (d, 1H), 7.19-7.10 (m, 1H), 7.09 (dd, 1H), 7.00 (d, 1H), 6.88 (t, 1H), 6.68 (d, 1H), 6.10 (d, 1H), 5.85-5.75 (m, 1H), 5.58 (dd, 1H), 5.06-4.89 (m, 4H), 4.29-4.17 (m, 2H), 3.38 (td, 2H), 2.41-2.34 (m, 4H), 2.20-2.11 (m, 2H), 1.91-1.87 (m, 2H), 1.30 (s, 3H), 1.22 (s, 3H);

- Figures 9A and 9B relate to polysiloxane matrices functionalized with SP molecules; Figure 9A shows a mechanochromic averaged activation pressure bubble chart, for all the silicone tested at different percentage by weight of SP doping; the size of the bubble radius and the colour legend represent the value of the minimum mechanochromic activation pressure; Figure 9B shows the numerical data corresponding to Figure 9A;

- Figures 10A, 10B, 10C and 10D show the normalized reflectance peak intensity at varying indentation force for each composition of PDMS matrices functionalized with different %wt of spiropyran molecules;

- Figures 11A, 11B, 11C and 11D show the reflectance spectra peak of mechanochromic activated coloration for each composition of PDMS matrices functionalized with different %wt of spiropyran molecules;

- Figure 12 shows the absorbance spectra of the SP form dissolved in dichloromethane, and of MC;

- Figures 13A, 13B relate to a thin film of 2%wt. SP-doped PDMS (2.5:1) and show the recovery time of the thin film at room temperature, under ambient light and under green diode laser light, respectively; Figure 13C shows the decay time characterization for all the PDMS samples at varying %wt. of SP doping concentration. The reflectance peak value at 480 nm on the value relative to the dominant wavelength (around 580 nm or 650 nm) of the sample varying on the time reports an exponential decreasing trend fitting;

- Figures 14A, 14B, 14C, 14D, and 14E show the modified da Vinci Research Kit (dVRK) platform employed for carrying out the experimental tests 1 and 2 as disclosed in the present application;

    the top panel of Figure 14A shows the master side and the slave side of the dVRK platform; the top panel of Figure 14B details the slave side of the dVRK platform; the bottom panels of Figures 14A and 14B show the key components and a side view, respectively, of a mechanochromic grasper as add-on onto a dVRK tool (Endowrist Cadiere Forceps), clutching a soft sample; the side view in the bottom panel of Figure 14B highlights the pressure transmission elements;

    Figure 14C shows the teleoperated mechanochromic grasper clutching a soft sample at 5 N, prior to mechanochromic activation (left panel) and after mechanochromic activation (middle panel), and the activated spot highlighted by removing the covering Plexiglas plate (right panel);

    Figure 14D shows the mechanochromic grasper clutching a soft sample at 7 N (by directly pulling tool cables), prior to mechanochromic activation (left panel) and after mechanochromic activation (right panel);

    Figure 14E shows an endoscopic image of the activated mechanochromic grasper, as acquired through the endoscopic camera natively integrated in the dVRK platform;

- Figure 15 shows the activation of the mechanochromic indicator onto a grasper according to a further embodiment of the invention. The figure also shows colour persistence over time when an object has to be retracted, by making a secure grip. The system subjected to pressure for a long time retains the colour; only the use of external green light or thermal relaxation of the polymer chains (i.e. toward thermodynamic equilibrium from a meta-stable equilibrium) bring the system back to the native color of the silicone.

**Detailed description of preferred embodiments**

In the following, several embodiments of the invention will be described. It is intended that the features of the various embodiments can be combined, where compatible. In general, subsequent embodiments will be disclosed only with respect to the differences with the previously described ones.

With reference initially to Figures 1A and 1B, a mechanical manipulation device, or end-effector, for use in Minimally-Invasive Surgery (MIS) according to a preferred embodiment of the present invention is globally denoted by 1. The end-effector 1 comprises two proximal jaw devices, reciprocally movable one toward the other to grasp a tissue between them. For the sake of simplicity, in the figures only one grasping jaw device is shown, namely that represented as the upper one, and denoted by 100.

The grasping jaw device 100 comprises a proximal jaw 2, in particular an adaptively morphing soft jaw, or morphing jaw, preferably implemented as disclosed in WO2019155316A1.

Morphing jaw 2 is associated, in particularly fixedly connected, to a dVRK (da Vinci Research Kit) tool jaw, or arm, 4. In the present example, the connection is obtained by a mechanical joint implemented by a screw 8 fitting into a seat 20 of the jaw 2 and a windowed area 40 of the arm 4 and by an associated closing nut 9.

The lower jaw device (not represented) may have the same structure as the upper jaw device, also as associated to another dVRK arm.

For better clarity, in the following description directions will be defined in conjunction with a reference system (*x, y, z*) fixed with jaw 2.

In particular, the end-effector 1 mainly develops along a longitudinal direction substantially parallel to the *x* axis.

Upper jaw 2 is rotatably coupled to the lower jaw according to a transverse rotation axis, the latter being parallel to the y axis and orthogonal to longitudinal direction x. Therefore, the proximal jaws close towards each other according to a closing direction which generally corresponds to the z axis.

The upper morphing jaw 2 is exemplified in more detail in Figures 1C and 1D.

With main reference to these latter figures, the jaw 2 includes a front element 101a that, in operation, is in contact with a tissue T to be grasped. Front element 101a faces the lower jaw and grasps tissue T therebetween.

Jaw 2 also comprises a back support element 102a configured to receive a grasping force which is exerted distally by the operator through a distal command and transmitted to support element 102a as grasping force $F_z$, i.e. a force in the mutual closing direction of the proximal jaws. Both the front element 101a and the support element 102a develop according to a main longitudinal direction parallel to the *x* axis, and to a secondary transversal direction parallel to the y axis.

Front element 101a and support element 102a are tightly coupled together. In particular, they are fixed one another along respective longitudinal edge portions so as to define a tubular hollow structure therebetween.

More particularly, both elements 101a and 102a have a curved profile and, in the present example, define a jaw which, in cross-section, is substantially shaped as an inverted "ω" closed at the middle or an overall heart shape. Therefore, jaw 2 has a general tubular configuration, with two longitudinal rooms or spaces 151 and 152 extending therethrough. Preferably, these rooms and spaces house elastomeric material.

Preferably, each element 101a, 102a is shaped, in cross-section, as a double arch.

In particular, front element 101a can be shaped substantially as an "ω". In other words, front element 101a has a substantially bilobed or "lowercase omega" profile. It has two lobes or coves 120, 121, preferably symmetric, arranged side by side. Thus, an end of a lobe is conjunct with an end of the other lobe at an intermediate region 122. The two lateral free ends of lobes 120 and 121 terminate with a lateral abutment or bracket 123, 124 protruding, along the y axis, inwardly with respect to the space subtended by each lobe 120, 121. Preferably, such lateral abutments 123 and 124 are substantially flat in the longitudinal direction x.

Support element 102a is defined by two arched sections 131, 132, in particular defining a continuous arched profile with a single radius of curvature. Support element 102a has at a central, or intermediate, rib 135 protruding, along the z axis, inwardly with respect to the space subtended by the arched profile, substantially centrally with respect to the circumferential development of the arched profile. Each of the two opposite ends of the arched profile terminates with a lateral abutment or bracket 133, 134 protruding, along the y axis, inwardly with respect to the space subtended by the arches. Preferably such lateral abutments 133 and 134 are substantially flat in the longitudinal direction x. Front element 101a and support element 102a are fixed one another along the respective lateral abutments 123, 133 and 124, 134, representing their longitudinal edge portions. In addition, elements 101a and 102a are coupled together at rib 135 and

corresponding region 122.

Front element 101a and support element 102a are elastically deformable. In particular, front element 101a is more compliant than support element 102a.

The overall arrangement is such that, by virtue of the proposed configuration - in particular of material continuity and compliance - when a grasping force $F_z$ is applied along the z axis to support element 102a, the latter deforms accordingly, in particular through a displacement along the y axis. The grasping force is as well transmitted to the front element 101a that, in turn, deforms along the y axis.

Figures 1C and 1D also depict schematically the contact area $A_1$ and $A_2$, respectively, between front element 101a and tissue or organ T being grasped. In particular, Figures 1C and 1D show an unloaded and a bent, or deformed, configuration, respectively, of the jaw 2.

As shown in Figure 10, in presence of grasping force $F_z$ support element 102a and front element 101a adaptively deform along the transversal direction y (displacement $d_y$), so that the contact area A between the front element 101a and the tissue T increases ($A_2$>$A_1$). A further increase in $F_z$ would induce a further increase in the contact area A, up to a limit configuration. Thus, grasping force $F_z$ is effectively transmitted to tissue T, but the resulting mechanical stresses distribute over an extended contact area $A_2$.

Then, upon removal of the grasping force $F_z$, each jaw goes back to its original "unloaded" configuration thanks to its elastic behaviour.

Grasping is effective and safe since, by design, the contact area between each front element 101a and the tissue T adaptively increases with the grasping force in such a way that a firm retention is provided without damaging the tissue. In other words, the deformation of the support element and of the front element, as response to the grasping force, determines an increase in the contact area between the front element and the tissue. Consequently, the force applied to the tissue is redistributed over a larger contact area so that the stress threshold is reached at higher force level with respect to the prior art devices.

With main reference again to Figures 1A and 1B, according to the invention the end-effector 1 comprises a mechanochromic indicator, or sensor, 3. In the present embodiment, indicator 3 is placed distally with respect to proximal jaw 2, and in particular upon the dVRK arm 4.

Preferably, indicator 3 is arranged between a transparent top plate 5 and a bottom transparent plate 6 associated with the dVRK arm 4, on the upper/external surface of the latter.

Transparent top plate 5 comprises pressure means 10, in particular one or more indenting elements or indenters, or relief structures, protruding downward so as to abut indicator 3. As it will be explained shortly below, pressure means 10 is responsible for the colour change of the mechanochromic indicator 3.

Preferably, the pressure means 10 is configured so as not to perforate or provide a preload in the steady-state to the mechanochromic indicator 3.

In the present example, device 1 includes one indenter 10 in form of a pillar, preferably a cylindrical one. The indenter may also be in form of a slab. As mentioned, the indenter(s) may vary in number as well as in thickness and/or width.

When the indenter 10 is a pillar, preferably it has a thickness of about 0.5 - 1.5 mm and a width of about 1.19 - 5.00 mm.

When the indenter is a slab, preferably it has a thickness of about 0.5 mm - 2.0 mm and a width of about 1.0 mm - 5.0 mm.

The indenter 10 is optically transparent. Preferably, its Young modulus is comprised in a range of about 5 MPa (e.g. Flexible 80A® Formlab) - 3 GPa (e.g. Plexiglas) or 5 MPa -10 GPa.

Suitable materials for the indenter 10 may be transparent to allow visibility from the top side view, for example polyvinyl chloride (PVC), polyethylene (PE), polylactic acid (PLA), polyethylene terephthalate (PET).

A spacer element 7 is arranged laterally to indicator 3.

The transparent top plate 5 is mechanically connected to the dVRK arm 4, the morphing jaw 2 and the spacer element 7 by means of the screw 8 and nut 9. Of course, other fixing means may be used. In Figure 1B, reference numbers 70 and 50 denote suitable seats obtained in the spacer 7 and transparent top plate 5, respectively.

Therefore, a stack is defined which comprises, starting from a basal plane, the head of the screw 8, the dVRK arm 4 at windowed portion 40 thereof, the morphing jaw 2 at seat 20 thereof, the spacer element 7 at seat 70, the transparent top plate 5 at seat 50 and the nut 9.

In specific implementations, the screw head has a preferred diameter of about 2.65 mm and the windowed area of the arm 4 preferably has an opening of about 2.55 mm.

The mechanochromic indicator 3 may be in the form of a brick, for example with size $(4\times5\times2)\ mm^3$. Its thickness along the z direction may be included in a range of 0.5-2.0 mm. The upper limit (maximum) of the thickness may vary in dependence of the thickness of the spacer element 7.

Preferably, the screw head is not fully tightened with the nut 9.

As it will be described in greater detail shortly below, the upper jaw 2, the arm or dVRK jaw 4, the screw 8 and

the plate 5 implement a lever transmission mechanism that transfers a contact force between the jaw 2 and the tissue T as a compression force exerted by indenter 10 onto indicator 3. Such lever mechanism, in the present example, is a first-type lever. Advantageously, it has a fulcrum at the longitudinal axis of the screw 8, which is preferably mounted with backlash in respective seat 20 of jaw 2, and pivots about an axis $P_y$ substantially parallel to the y direction.

Variant embodiment may provide for indicator 3 to be arranged directly onto proximal jaw 2. The indicator 3 may also be provided upon both the upper and lower jaw, or at both facing sides of the end-effector 1.

The mechanochromic indicator 3 will now be disclosed in greater detail.

Preferably, indicator 3 provides a reversible colour change, detectable by naked eye, when subject to a triggering force.

In the present embodiment, indicator 3 comprises at least one polymeric matrix functionalized with mechanochromic molecules.

In particular, a prepolymer/curing agent weight ratio used for preparing said at least one polymeric matrix is comprised in the range of about 1:1 - 15:1.

Preferably, the percentage by weight of the mechanochromic molecules in the matrix is comprised in the range of about 0.1 - 5%, more preferably is comprised in the range of about 1-5%.

As used herein, the term "polymeric matrix" means a tridimensional polymeric network.

The term "prepolymer", as used herein, refers to materials which polymerize *in situ* to form a polymeric network and may encompass monomers, oligomers, short chain pseudo-stable polymeric chains which are capable of further polymerization by reactive groups to a fully cured high molecular weight state. As such, mixtures of reactive polymers with un-reacted monomers may also be referred to as pre-polymers. The terms "prepolymer" and "polymer precursor" may be interchanged.

The terms "curing" or "cure" refer to the chemical process employed in polymer chemistry that produces the toughening or hardening of a polymer material by cross-linking of the polymer chains. During the curing process, single monomers and oligomers react to form a tridimensional polymeric network.

Means for the fabrication of a polymeric matrix functionalized with mechanochromic molecules are well-known and commonly used in the field of polymer chemistry.

In one embodiment, the mechanochromic indicator 3 is prepared by a curing reaction of a composition comprising a prepolymer and mechanochromic molecules in the presence of a curing agent, provided the prepolymer/curing agent weight ratio is comprised in the range of 1:1 - 15:1, and the percentage by weight of said mechanochromic molecules in said composition is comprised in the range of 0.1 - 5%.

The term "curing agent", as used herein, refers to any material or combination of materials that initiate, propagate, or accelerate cure of the polymer matrix and includes but is not limited to accelerators, catalysts, initiators, and hardeners. Any suitable curing agents can be used to produce a polymeric matrix according to the present invention. Suitable curing agents may contain reactive functional groups that are reactive with the functional groups in the polymer matrix. Examples include, but are not limited to Platinum(0)-1,3-divinyl-1,1,3,3-tetramethyldisiloxane complex solution for hydrosilylation reaction (Karstedt's catalyst). More in general platinum, palladium, rhodium (Wilkinson's catalyst), and ruthenium form highly active catalysts; non-precious metal catalysts, especially those based on nickel have also been developed as economical alternatives, but they are often slower or require higher temperatures or typical strategy for the preparation of thermoset polymers, and by means of free radical polymerization.

In one preferred embodiment, the prepolymer/curing agent weight ratio used for preparing said at least one polymeric matrix of the mechanochromic indicator may be 1:1, 2.5:1, 5:1, 10:1, 15:1.

The curing reaction can be carried out by any technical means used in the art, under conditions suitable to produce a three-dimensional polymeric network functionalized with said mechanochromic molecules. Depending on the components selected for the preparation of the mechanochromic indicator of the present invention, the composition comprising the prepolymer and mechanochromic molecules may be cured at temperatures ranging from 90 °C and 150°C.

The matrix may be selected, preferably but not limitedly, from the group comprising the following polymers, alone or in combination: polydimethylsiloxane (PDMS), Dragon Skin 20®, poly(methylacrilate) (PMA), polycaprolactone (PCL), Ecoflex®, polymethyl methacrylate (PMMA), polystyrene and poly(n-butyl acrylate) triblock copolymers ((PS)-PnBA-SP-PnBA-PS),), ureidopyrimidinone (UPy).

The mechanochromic molecules may consist of molecules showing mechanochromism or mechanoluminescence, i.e. molecules that undergo a change of colour when exposed to mechanical stimuli.

More in detail, the mechanochromic molecules may be chosen, preferably but not limitedly, among functionalized spiropyran (SP) molecules and/or derivatives thereof (e.g. spirothiopyran, naphthopyran, bis-naphthopyran), polydiacetylenes, azo moieties, rhodamine and/or derivatives thereof (e.g. spirorhodamine-diol), benzocyclobutene, diarylbibenzofuranone moieties, perylene bisimide, 1,2- dioxetane, oligourethane gel, phenyltriazolinedione-anthracene, coumarin dimers, dithiomaleimide moieties, methanone-tethered cinnamate dimers, 2-(20-hydroxyphenyl)-benzoxazole, silver coordination complexes, cyano-OPV derivative, bis(benzoxazolyl)stilbene dye, Square-planar platinum(II) complexes, 2-Phenyl-1,3-indandione radical and its derivatives, 1,1,1-tris(cinnamoyloxymethyl)ethane, 1,4-bis(R-cyano-4-methoxystyryl)-2,5-dimethoxybenzene (BCMDB), 1,4-bis(R-cyano-4-meth-oxystyryl)benzene (BCMB), carbazole units, poly(3-alkylthiophene), Rotaxanes), and/or combinations thereof.

The amount of the mechanochromic molecules in the at least one polymeric matrix may be adjusted depending on the type of polymer and mechanochromic molecules used, provided it can ensure a detectable mechanochromic response when the resulting mechanochromic indicator is subjected to a minimum activation pressure. Preferentially, the mechanochromic molecules may be present in the at least one polymeric matrix in a percentage by weight of 0.1%, 0.5%, 1%, 1.5%, 2%, 4% or 5%. Within the context of the present invention, the terms "doped" or "doping" can be used with reference to a polymeric matrix (or the resulting "mechanochromic indicator") that is functionalized with mechanochromic molecules according to any of the embodiments as described in the present specification. As an example, the terms "SP-doped" or "SP-doping" can be used to define a polymeric matrix that is specifically functionalized with spiropyran molecules or derivatives thereof such as those described in the present specification acting as cross-linker or coupling agent in the polymer network chain.

In some embodiments, the mechanochromic molecules form or are incorporated within a coating on the surface of said at least one polymeric matrix and/or are incorporated within the polymeric structure of said at least one polymeric matrix.

Preferably, the mechanochromic molecules are covalently linked to said at least one polymeric matrix: in particular, such mechanochromic molecules may be covalently integrated into the main chain (molecular backbone) and/or into a side chain of said polymeric matrix, and/or may form a side chain of said polymeric matrix. Merely by way of example, mechanochromic molecules can be incorporated in the polymer chain with a chain-growth, or step-growth polymerization procedure, or with post-polymerization coupling steps (e.g. Diels-Alder reactions). Furthermore, the former can be incorporated also at the interface (immobilizing the mechanophore on the surface), or with, for example, sol-gel strategies and covalent attachment of the dye to micelles. Preferentially, molecules of the polymeric matrix can be crosslinked with one another *via* said mechanochromic molecules.

In one embodiment, said at least one polymeric matrix may be characterized by a porous architecture or else by a nanoparticle-in-micropore (NP-MP) architecture comprising mechanochromic molecules and nanoparticles (e.g. silica nanoparticles). One example of such mechanochromic composite with hierarchical NP-MP architecture is provided in Park et al. 2019, Adv. Mater 2019, 31, 1808148.

Merely by way of example, porous mechanochromic indicators with hierarchical NP-MP architectures can be fabricated by mixing said mechanochromic molecules (e.g. spiropyran molecules) with a PDMS polymer and silica nanoparticles in hydrophilic cosolvents, followed by the evaporation of solvents during the hydrosilylation curing process. During this process, spherical pores are formed within the composites as a result of phase separation between the hydrophobic PDMS matrix and hydrophilic solvents, following the elimination of trapped solvents. The resulting composite or polymeric matrix incorporating said mechanochromic molecules, would contain uniformly distributed pores with inner pore surfaces decorated with silica nanoparticles.

Advantageously, hierarchical NP-MP architecture in porous mechanochromic indicators can enhance both mechanochromic strain/stress sensitivity and stretchability. Notably, reducing pore size while increasing nanoparticle size should enhance the mechanochromic sensitivity. Furthermore, the hierarchical NP-MP architecture of the porous polymeric matrix can simultaneously reduce the onset of mechanochromism and enhance the extensibility.

In some embodiments, said mechanochromic molecules may be functionalized with one or more terminal functionalization groups suitable for their covalent binding to said at least one polymeric matrix. Means for the chemical functionalization of said mechanochromic molecules are well-known to those of ordinary skill in the art, who would be able to select the most suitable functionalization groups and functionalization protocols depending on the type of polymeric matrix and type of mechanochromic molecules that are selected.

Preferentially, said mechanochromic molecules are functionalized spiropyran molecules. A spiropyran is a 2H-pyran isomer comprising an indoline and a chromene moiety bound together via a spiro junction and oriented perpendicular with respect to one another. Colorless spiropyran reversibly isomerizes to the planar zwitterionic blue/violet-colored merocyanine (MC) form upon mechanical, optical or thermal stress. The MC $\rightarrow$ SP reverse isomerisation usually occurs spontaneously and can be accelerated by visible light (e.g. white and/or green light pumping) or thermal heating.

One preferred embodiment of the present invention refers to an engineered mechanochromic indicator as previously described, wherein said at least one polymeric matrix is a PDMS matrix functionalized with SP molecules, the prepolymer/curing agent weight ratio used for preparing said PDMS matrix is equal to 2.5:1, and the percentage by weight of said SP molecules in said at least one matrix is 2 wt%.

Preferentially, said SP molecules are specifically functionalized with two terminal alkene chains (vinyl groups), one attached at the benzopyran ring, and one at the indoline ring of each SP molecule, thereby enabling the cross-linking of the SP molecules to the elastomeric network of the polymeric matrix.

In one preferred embodiment, said SP molecules have the following formula

(I): 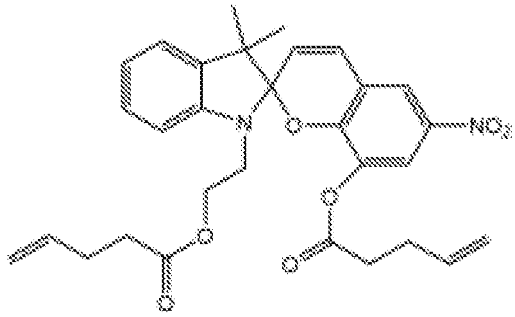

(I)

Merely by way of example, SP molecules bis-functionalized with alkene chains may be cross-linked into the elastomeric network of poysiloxanes by a platinum catalyzed hydrosilylation reaction. Thanks to the bis-alkene functionalization groups, this reaction reveals as the same curing reaction as that between vinyl- and hydrosilane functionalities in the SP-free silicone networks.

Notably, as also schematically depicted in Fig. 6, the macroscopic stretching of the polymer chains of the mechanochromic indicator thus obtained can result into the spiro C-O bond break and the transformation from the SP to the highly colored MC form. The reversibility and in particular the closure of the spiro C-O bond in the MC form, can be fostered by pumping light on the macroscopic colored form. Indeed, a broad absorbance peak around 560 nm is distinguishable in the MC absorbance spectrum, suggesting that green light or in general white light can foster the reversible SP formation, as well described also in literature.

According to one embodiment of the present invention, the mechanochromic indicator is characterized by a minimum mechanochromic activation pressure comprised between 900 kPa and 10 MPa.

In some embodiments, the mechanochormic indicator can be advantageously characterized by a minimum mechanochromic activation pressure lower than 900 kPa: such values might be obtained, for example, by introducing/forming nano- and/or micropores within the polymeric structure of said at least one polymeric matrix (e.g. by obtaining hierarchical NP-MP architectures) and/or by modulating the curing temperatures that affect the elastic behaviour of said materials. Indeed, as previously mentioned, the hierarchical architecture of porous polymeric matrices can favourably reduce the onset of mechanochromism.

Advantageously, as also clearly shown in Fig. 9B, the preferred composition of the mechanochromic indicator as described above (namely, PDMS 2.5:1, 2%wt. SP-doped) ensures a minimum averaged mechanochromic activation pressure of 1.17 MPa and the fastest recovery time under optical pumping (≈150 s), under 520 nm optical laser diode pump driven at 125 mA at room temperature.

As previously mentioned, another way to foster the macroscopic recovery time for the backward reaction (MC → SP), relies on mediation through thermal heating. In particular, as clearly shown in Fig. 7, even at 40°C the recovery time reduces three times respect to standard ambient temperature and pressure (20/25°C - 1 Atm). This result is important since the temperature of the internal human environment ranges from 35°C-37°C. Therefore, by merging an external perturbation of light and heat (e.g. human environment), it is possible to drive the backward reaction (MC→SP) towards faster recovery times, approaching the typical thresholds of electronic sensors counterparts.

A mechanochromic indicator according to the present invention may further comprise the combination of a plurality of polymeric matrices, each functionalized with different mechanochromic molecules, and/or comprising different weight percentages of mechanochromic molecules, and/or having different thicknesses, so as to form a multi mechanochromic material with proper minimum mechanochromic response when the minimum mechanochromic activation pressure is achieved.

In the context of the present specification, a mechanochromic indicator comprising the combination of at least two or more polymeric matrices, each functionalized with different mechanochromic molecules and/or comprising different weight percentages of mechanochromic molecules, and/or having different thicknesses, may be also defined as a "multicolor mechanochromic indicator" or else as a "multi-mechanochromic indicator". On the other hand, a mechanochromic indicator consisting of a single polymeric matrix functionalized with mechanochromic molecules according to any of the previously described embodiments, may also be herein defined as a "single-color mechanochromic indicator" or else "single-mechanochromic indicator".

According to one embodiment of the present invention, the mechanochromic indicator 3 comprises at least three bonded polymeric matrices each comprising a distinct weight percentage of said mechanochromic molecules and/or having a different thickness, even showing a different colorimetric response (i.e. SP in Poly(methylacrilate) (PMA) red, SP in PDMS violet, SP in Polycaprolactone (PCL) cyan).

Operation of manipulation device 1 will now be described in greater detail with reference to Figure 1E.

When device 1 is in an operating condition, the morphing jaw 2 exerts a clutching force on tissue and/or organ T under manipulation. The tissue and/or organ T will provide an equal and opposite force $F_c$ in response. This latter force is transmitted, via the lever mechanism disclosed above, to the indenter 10, which exerts a compression force upon the mechanochromic indicator 3.

In the present embodiment, thus, the lever mechanism works by means of a sort of fulcrum transmission transferring clutching force $F_c$ acting on the soft front jaw element 101a into an indentation force $F_i$ acting on the mechanochromic indicator 3.

In an embodiment, the lever arms interposed between the fulcrum and, respectively, the contact point between jaw 2 and tissue T and the contact point between indenter 10 and indicator 3 are of comparable length (denoted by *l* in Figure 1E). Therefore, for the aforementioned mechanical actions, $F_c = F_i$ and the clutching pressure $p_c$ (acting on the corresponding jaw area $A_c$) can be immediately linked to the indentation pressure $p_i$ (acting on the corresponding indenter area $A_i$) as follows:

$$p_i \cong p_c\,(A_c/A_i).$$

This latter relation permits to regard the $A_c/A_i$ as a "geometrical amplification factor" for mapping a working pressure $p_c$ into a pressure $p_i$ suitably above the mechanochromic activation threshold of indicator 3. In a clinical perspective, the clutching pressure should be kept below a tissues-specific damage threshold (e.g. in a range of about 140-240 kPa for porcine organ models).

The transmission of force $F_c$ is allowed, in the present embodiment, by a backlash between the seat 20 of the morphing jaw 2 and screw 8.

Such backlash allows to the morphing jaw 2, in response to the force $F_c$ provided by the tissue and/or organ under manipulation, to tilt outwards with respect to its longitudinal axis along the *x* direction, consequently tilting the spacer element 7 and the transparent top plate 5, so that the indenter 10 may exert a compression force $F_i$, equal in value to the force provided/received by the morphing jaw 2, on the mechanochromic indicator 3.

When the indenter 10 exerts a compression force on the mechanochromic indicator 3, this result in a colour change. The device 1 is configured so that the colour change takes place when the force transmitted reaches a value that is equal or just below the tissue and/or organ damage threshold.

The indenter 10 may exert compression forces as low as 1N.

The colour change can be visualized by the surgeon by suitable vision means. According to a first preferred embodiment, the surgeon may notice the colour change by means of an endoscope, which is commonly used during MIS/MIRS procedures.

As exemplified in Figures 2A to 2C, according to a second preferred embodiment, alternative or combinable with the previous one, the colour change, and more in particular the intensity of such colour change, may be detected by means of at least one optical fiber 200.

This is particularly useful when the mechanochromic indicator comprises two or more matrices, each one loaded with different percentage by weight of SP molecules. Preferably, the optical fiber is inserted in a concentric hole in one indenter 10. In the variant embodiment of Figure 2A, six indenters are provided. The optical fiber can be posed inside the indenter, recording the colorimetric spectroscopic data as depicted in the exemplary plot of Figure 2C.

More preferably, each indenter provides a concentric hole suitable for housing a respective optical fiber.

The optical fiber may consist in a poly(methyl methacrylate) (PMMA) optical fiber. Preferably, the fiber minimum diameter is as low as 0.5 mm, and it is strictly dependent on the indenter diameter in which the optical fiber will be housed into.

As exemplified in Figure 2C, the optical fiber 200 may be connected outside with a light source and a spectrophotometer. Such system can derive colorimetric measurements, returning the different spectra at different indenting forces. This second embodiment introduces the wiring of at least an optical fiber along the indenter profile, and it can pass along a support shaft of the manipulation device without hampering the actuation mechanism or the biocompatibility of the structure.

Regardless the means used for detecting the colorimetric variation, that is by using the endoscope or the optical fiber, when the colour change takes place, the surgeon can choose to stop engaging the tissue and/or organ T.

Without any clutching force exerted, the colour change of the mechanochromic indicator may be reversed. Such reversibility may occur by waiting for a certain amount of time, called herein recovery time.

Preferably, such recovery time may be fostered by suitable means, such as by pumping light or providing heat transfer to the mechanochromic indicator. According to a preferred embodiment, the colour change may be reverted by a white light and/or green light pumping. Such pumping is preferentially provided by the endoscope, which is equipped with a light source for the lighting of the surgical area.

As exemplified in Figure 3, an inspection endoscope 400, which accompanies the manipulation returning a 3-D fully coloured view to the surgeon, may also be used for fostering the reversibility of the colour change.

The endoscope may, at the same time, alarm the surgeon when a colour change on the end-effector takes place as well as promote the starting colour recovery of the mechanochromic indicator by pumping light over the end-effector.

In specific embodiments such recovery time may vary in the range 900 s (worst case at room temperature under optical pumping with green laser driving current 125 mA for PDMS 5:1, 5%wt. SP-doping) - 2 s (best case at 100 °C under ambient light for PDMS 2.5:1, 2%wt. SP-doping).

Preferably, in specific embodiments such recovery time may vary in the range 245 s (worst case at room temperature and with ambient light for PDMS 2.5:1, 2%wt. SP-doping) - 40 sec (best case at 40°C under optical pumping with green laser driving current 125 mA for PDMS 2.5:1, 2%wt. SP-doping). Theoretical values of the recovery time could be specifically evaluated by scaling the value obtained passing from 20/25°C to 40° under ambient light, by a factor of 3.

According to a variant embodiment, the mechanochromic indicator can be encapsulated.

According to an embodiment shown in Figure 4, the pressure means, here denoted by 110, has a plurality of indenter elements for obtaining, in particular imprinting, a visual message upon the indicator 3, e.g. an alphanumeric message like "STOP".

Both pressure means 110 and an associated lever mechanism for force transmission can be directly 3D printed, e.g. by commercial photo-curable resins.

According to a further embodiment, a multi-colour mechanochromic sensor, or indicator, can be realized, miniaturized, and easily laid down on the surgical tool frame as demonstrated for the previously described single-colour mechanochromic indicator. Indeed, covalent binding of SP molecules in e.g. Poly(methylacrilate) (PMA), polycaprolactone (PCL), or polysiloxanes (PDMS, Dragonskin®, Ecoflex®, etc.), results in a mechanochromic polymer with a visible absorbance spectrum in the open merocyanine form ranging from reddish, cyanic, and intense blue/violet, respectively. Advantageously, multi-colour and tunable colorimetric information from different polymers functionalized with different %wt. of SP molecules could be useful for applications requiring spatial-resolution or multiple-activation-thresholds.

As exemplified in Figures 5A and 5B, a multi-mechanochromic structure can be easily bonded, resulting in a sensor, or indicator, 30 for different safe damage tissue/organs pressure threshold (three in the example shown). In particular, Figure 5B shows three mechanochromic elements (MCE) with different exemplary activation force thresholds. In one embodiment, said multi-mechanochromic indicator may comprise different polymeric matrices specifically functionalized with different percentages of SP molecules, acting as discrete colorimetric pressure sensors with a proper minimum mechanochromic activation pressure.

In an embodiment, pressure means 10 with multiple indenting elements can be used in order to selectively transfer the reaction force generated during the engaging of the jaw with the tissue at the different mechanochromic polymers positions.

In this way, activation of each of this SP-doped family of polymers with different mechanochromic activation pressure, can return a selective alarm to the user for different tissues thresholds and for real-time dynamic manipulation tasks. Indeed, the lever mechanism would trigger a different mechanochromic response when the minimum mechanochromic pressure is achieved.

The present disclosure provides also a method of operation of a mechanical end-effector as disclosed above, which method comprises the following steps:

(a)applying a compression force to a tissue and/or organ by means of the mechanical end-effector;

(b)detecting a colour change of the mechanochromic indicator by means of at least an endoscope;

(c) releasing the tissue and/or organ under manipulation;

(d)preferably, providing at least an optical pumping for a certain amount of time by means of the endoscope for reverting the colour change of the mechanochromic indicator;

(e)applying again a compression force to the same tissue/and or organ or to another tissue and/or organ by means of the mechanical end-effector.

Optical pumping according to step (d) may be omitted in specific applications, wherein colour reverting occurs spontaneously over time.

Examples are reported below, which have the purpose of better illustrating the methodologies disclosed in the present description; such examples are in no way to be considered as a limitation of the previous description and the subsequent claims.

## Examples

Mechanical characterization

Different silicone-based matrices have been mechanically tested using a Universal Testing Machine (UTM). Each prepolymer to curing agent ratio was identified by the relative elastic modulus. The elastic moduli by monoaxial stress-strain traction tests on standardized dog-bone silicone not doped with SP molecule was firstly assumed (Table 1). The mechanical performances of PDMS is affected by different percentage of the cross-linker sites, and it has been proved that a higher number of cross-linker sites results in a higher Young modulus of the material.

Table 1: a) All the sample have been cured at around 90°C per 5 h except c); b) All the measurements have been repeated at least five times on different cured dog-bone samples; c) cured at 150°C per 2 h

| Material Type | Curing ratio (prepolymer : catalyst) | Elastic Modulus [MPa][a) b)] | Standard deviation [MPa] [b)] |
|---|---|---|---|
| PDMS | 2.5:1 | 4.79 | 1.18 |
| PDMS [c)] | 2.5:1 | 21.93 | 1.44 |
| PDMS | 5:1 | 3.26 | 0.10 |
| PDMS | 10:1 | 2.29 | 0.04 |
| PDMS | 15:1 | 1.97 | 0.16 |
| PDMS | 40:1 | 0.54 | 0.17 |
| Dragonskin-20 | 1:1 | 0.78 | 0.24 |

Preparation of polymers functionalized with spiropyran molecules (SP)

Samples were fabricated by reticulating different elastomeric materials as Polydimethylsiloxane (PDMS, Sylgard 184), and Dragonskin20 (Smooth-On®) with the SP monomer, doubled-functionalized with terminal vinyl groups. Doubled-functionalized SP molecules can be synthesized according to the following reaction scheme:

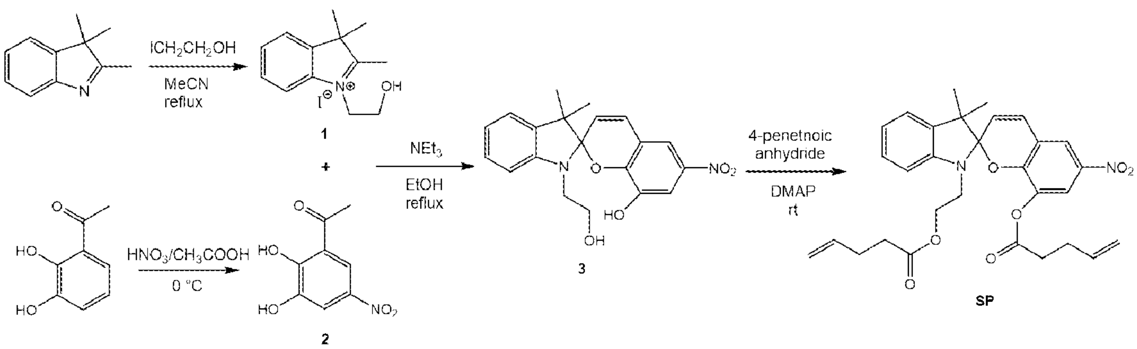

1
2
3
SP

Scheme 1: Chemical schematic.

Details on the chemical syntheses and sample fabrication can be found elsewhere (G. R. Gossweiler, G. B. Hewage, G. Soriano, Q. Wang, G. W. Welshofer, X. Zhao, S. L. Craig, ACS Macro Lett. 2014, 3, 216.). Hydroxyl-functionalized SP molecules (3) were purified by column chromatography, and the synthesis and purification of the final product (SP) were performed according to the procedure by G. R. Gossweiler, T. B. Kouznetsova, S. L. Craig, J. Am. Chem. Soc. 2015, 137, 6148. However, column chromatography ($Al_2O_3$ neutral, dicloromethane DCM, Sigma Aldrich) was used to purify the final product. All the chemical compounds were verified by 1H-NMR and 13C-NMR tests, with a Bruker 400 MHz spectrometer (Bruker) at 300 K (Figure 8).

The needed weight fraction of the bis-functionalized SP monomer was put into a glass vial and dissolved in 1.2 ml of DCM. The solution was mixed with the PDMS, or Dragonskin20 pre-polymer and stirred vigorously for 1 h. After 1h, the curing agent was added to the mixture and stirred. After another hour, the solution was poured into a rounded mold. It was degassed until all the DCM fraction was evaporated and all the poured molds were put in the oven at 90°C until the curing process was ended (around 9 h). The mechanochromic samples thus obtained varied their colour from yellowish (small mg amount of SP) to dark-orange coloration (high percentage by weight of SP).

Characterization of the mechanochromic indicators

All the compression simulations were carried out by using FEM solver Abaqus (Dassault Systemes, Vélizy-Villacoublay, France). During the simulation, the material model adopted for the pillar and slab is a linear elastic plexiglass, with an equivalent Young Modulus being 3 GPa and Poisson's ratio being 0.37. The material model adopted for the mechanochromic indicator is a built-in hyperelastic Neo-Hookean model with material constants C10 being 0.798 MPa and D1 being 0. Standard 3D stress elements were adopted, as well as inviscid contact. Grid independence was checked. The interaction property between the touched surfaces only accounts for normal behaviour. The plexiglass components are laser-cutted by Versalaser machine.

The macroscopic mechanochromic response of a series of PDMS (2.5:1, 5:1, 10:1, 15:1, prepolymer to curing agent ratio), and Dragonskin20 (1:1) rounded samples, doped with different percentage by weight of SP as previously synthetized (1%, 1.5%, 2%, 4% and 5%) and cured at 85°C-95°C was investigated. The opto-mechanical response of the selected doped elastomers when transiently subjected to a mechanical compression, in order to derive the minimum mechanochromic activation pressure and the visibility of the changing coloration, has been deeply characterized.

As clearly shown in Figure 9B, a minimum activation pressure of around 926 kPa (averaged activation mechanochromic pressure is 1.17 MPa) was obtained for the optimal composition of PDMS (2.5:1) with 2% wt. SP-doping.

Concerning the optical response of the mechanochromic indicators, the normalized ratio value (N [R ($\lambda$ = 480 nm) / R ($\lambda$0)]) was plotted in function of the force (Figure 10). The graphs reported in Figures 10A-D specifically illustrate the reflectance peak intensities at varying indentation force obtained for different PDMS matrices (characterized by the following prepolymer to curing agent ratios: 2.5:1, 5:1, 10:1, 15:1, respectively) functionalized with a different percentage by weight of SP molecules as previously synthetized (namely 1%, 1.5%, 2%, 4% and 5%).

R ($\lambda$ = 480 nm) represents the reflectance peak of the merocyanine form at 480 nm, and R ($\lambda$0) (where $\lambda$0 is equivalent to 580 nm for all the prepolymer/curing agent compositions except for the 15:1 that shows a broad peak at 650 nm) was the reflectance peak at the dominant yellowish/reddish sample coloration. The straight line set in each graph reported in Figures 10A-D is a naked-eye threshold decided in order to discriminate whether the changing of coloration is evident.

The graphs reported in Figures 11A-D correspond to the reflectance spectra obtained at varying indentation force for each of the above-mentioned PDMS samples. Furthermore, the recovery time of the mechanochromic indicators was tested, i.e. the recovery time from the backward chemical reaction MC->SP. Indeed, while the changing coloration reaction SP->MC is instantaneous when reached the mechanochromic activation pressure, the backward shows a time latency. The reversibility and in particular the closure of the spiro C-O bond in the MC form, can be fostered by pumping light on the macroscopic colored form. Indeed, a broad absorbance peak around 560 nm is distinguishable in the MC absorbance spectrum (Figure 12), suggesting that green light or in general white light can foster the reversible SP formation, as well described also in literature.

Figure 13A and Figure 13B relate to a thin film of 2%wt. SP-doped PDMS (2.5:1) and show the recovery time of the thin film at room temperature, determined under ambient light and under green diode laser light, respectively.

Figure 13C shows the decay time characterization for all the PDMS samples (characterized by the following prepolymer to curing agent ratios: 2.5:1, 5:1, 10:1, 15:1, respectively) at varying %wt. of SP doping concentration. The reflectance peak value at 480 nm on the value relative to the dominant wavelength (around 580 nm or 650 nm) of the sample varying on the time reports an exponential decreasing trend fitting.

The composition of PDMS 2.5:1, 2%wt. SP-doped, reported the minimum mechanochromic activation pressure (1.17 MPa) and the fastest recovery time under optical pumping ($\approx$150 s) of all sample tested, under 520 nm optical laser diode pump driven at 125 mA at room temperature.

Another way to foster the macroscopic recovery time for the backward reaction (MC->SP), relies on mediation through thermal heating. The sample was placed in an oven at uniform temperature. The sample was then manually indented, reproducing a circular-coloured region and with a digital timer, the time recovery was measured. As clearly shown in Figure 7, even at 40°C the recovery time reduces three times respect to standard ambient temperature and pressure (20/25°C - 1 Atm).

Experimental operating tests using a manipulation device according to an embodiment of the present invention

Test 1

A first experimental test was performed by manually actuating an Endowrist Cadiere Forceps (by Intuitive Surgical, product number 400049), modified so as to incorporate (see Figures 14 A-E and 15):

- a mechanochromic indicator having the optimal composition of PDMS (2.5:1) with 2% wt. SP-doping, with averaged activation mechanochromic pressure of 1.17 MPa;

- suitable pressure means (i.e. cylindric indenter) and a force transmission mechanism according to the present invention, so as to transfer the load applied onto a tissue on the mechanochromic indicator.

Measurements of the force applied onto the tissue have been obtained by an electronic force sensor (FSR-402, Interlink Electronics, Inc., USA), that was sandwiched between the grasped soft tissue (see Figure 14A, bottom).
Thanks to the lever (amplification) mechanism, a 7N grasping force applied by the distal jaw to the tissue, with the contact area of around 100 $mm^2$, could be transferred to the proximal indenter to the mechanochromic indicator, with a contact area of 1.13 $mm^2$, allowing to implement a mapping between the tissue damage threshold pressure and the trigger pressure for change of coloration.
The amplification of the applied force allowed the color changing of the mechanochromic indicator to be confined on a very small area (cylindrical indenter area, 1.13 $mm^2$) achieving the high value of 1.2 MPa (out of scale of the surgical application). However, due to the fact that the soft grasper in contact with the soft tissue had a contact area of 100-120 $mm^2$, it has been possible to transfer a pressure on the soft tissue of about 0.07 MPa (order of surgical application, it can be a compatible/reliable damage threshold alert).

Test 2

A second experimental test was carried out endowing a da Vinci Research Kit (dVRK) (Intuitive Surg. Inc., CA, USA), by robotic actuation of an Endowrist Cadiere Forceps (by Intuitive Surgical, product number 400049) modified with the technical elements as described above for the first experimental test, so as to transfer the load on the mechanochromic indicator. The color changing was obtained at 5 N on the soft grasper. However, the available dVRK surgical robotics platform permits to clutch with forces up to nearly 6 N. Moreover, considering that the used dVRK platform was a first-generation system, it was not possible to exert clutching forces as high as those available on current da Vinci clinical systems (e.g., da Vinci S, Si, and Xi), nor it could be possible to benefit from higher resolution currently available for its endoscopic camera. Nonetheless, the obtained results demonstrate the possibility to integrate a mechanochromic grasper in a state-of-the-art surgical robotic system, by leveraging material intelligence to achieve sensing capabilities without resorting to extremely complex electronics implementations). These forces were distributed on the soft grasper area (100-120 $mm^2$) providing 0.05 MPa on the soft tissue. The latter pressure is very low compared to the mechanochromic activation pressure threshold (1.17 MPa) but it is amplified, providing the required color changing to alarm the surgeon.
The present invention has been described with reference to preferred embodiments. However, it will be understood that variations and/or modifications can be brought to the grasper device according to the present invention without thereby departing from the scope of the invention as defined in the following claims.

## Claims

**1.** A manipulation device (1) for minimally-invasive surgery, comprising at least one grasping device (100) configured for acting upon a tissue,

which grasping device (100) comprises a mechanochromic indicator (3),
wherein said mechanochromic indicator (3) has at least one polymeric matrix functionalized with mechanochromic molecules,
wherein said mechanochromic indicator (3) is configured to provide the operator with a visual colour feedback when a pre-determined threshold force or pressure (- $F_c$, $p_c$) is applied upon the tissue,
wherein said manipulation device (1) further comprises a force transmission mechanism (8, 5) to transfer a reaction force ($F_c$) applied from the tissue upon a proximal jaw (2) of said grasping device (100) to said mechanochromic indicator (3),
**characterized in that** said manipulation device (1) further comprises pressure means (10) configured to apply a pressure upon said mechanochromic indicator (3),
wherein said force transmission mechanism is configured so that said pressure means (10) apply a pressure upon said mechanochromic indicator (3) which is amplified with respect to the pressure applied from the tissue upon said proximal jaw (2).

**2.** The manipulation device (1) according to claim 1, wherein said force transmission mechanism is a lever-type mechanism, preferably a first-type lever mechanism having a central fulcrum (8).

**3.** The manipulation device (1) according to claim 2, wherein said lever-type mechanism has a pivoting axis ($P_y$)

extending according to a pivoting direction (y) traverse to both a tissue force application direction (z) and a longitudinal prevalent direction of development (x) of said proximal jaw device (100).

**4.** The manipulation device (1) according to any one of claims 1 to 3, wherein said pressure means (10) applies a pressure upon a selected area of said mechanochromic indicator (3).

**5.** The manipulation device (1) according to any one of claims 1 to 4, wherein said pressure means comprises one or more indenter elements (10) protruding from a base member (5), said one or more indenter elements (10) being each preferably configured as a slab or pillar.

**6.** The manipulation device (1) according to any of claims 1 to 5, wherein said pressure means (110) are configured to imprint a visual message, in particular an alphanumeric message, upon said mechanochromic indicator (3).

**7.** The manipulation device (1) according to any one of claims 1 to 6, wherein the pressure $p_i$ applied upon said mechanochromic indicator (3) and the pressure $p_c$ applied upon said proximal jaw (2) are linked by the following equation:

$$p_i \cong p_c\ (A_c/A_i),$$

wherein $A_c$ and $A_i$ denote the pressure application area upon the proximal jaw (2) and the mechanochromic indicator (3), respectively.

**8.** The manipulation device (1) according to any of claims 1 to 7, comprising one or more transparent elements (5, 6, 10) to allow the operator to receive a visual feedback from said mechanochromic indicator (3).

**9.** The manipulation device (1), according to any of claims 1 to 8, wherein mechanochromic indicator (3) is shaped substantially as a brick.

**10.** The manipulation device (1), according to any of claims 1 to 9, wherein mechanochromic indicator (3) has a reversible colour change.

**11.** The manipulation device (1), according to any of claims 1 to 10, wherein said mechanochromic indicator (3) comprises at least one polymeric matrix functionalized with mechanochromic molecules, **characterized in that**:

- a prepolymer/curing agent weight ratio used for preparing said at least one polymeric matrix is comprised in a range of about 1:1 - 15:1; and
- a percentage by weight of said mechanochromic molecules in said at least one matrix is comprised in a range of about 0.1 - 5%.

**12.** The manipulation device (1), according to claim 11, wherein said at least one polymeric matrix is selected from the group comprising polydimethylsiloxane (PDMS), poly(methylacrilate) (PMA), polycaprolactone (PCL), polymethyl methacrylate (PMMA), polystyrene and poly(n-butyl acrylate) triblock copolymers ((PS)-PnBA-SP-PnBA-PS),), ureidopyrimidinone (UPy) and/or combinations thereof.

**13.** The manipulation device (1), according to claims 11 or 12 wherein said mechanochromic molecules are functionalized spiropyran (SP) molecules.

**14.** The manipulation device (1), according to any of claims 11 to 13, wherein said mechanochromic molecules are covalently linked to said at least one polymeric matrix.

**15.** The manipulation device (1), according to any of claims 11 to 14, wherein said at least one polymeric matrix is a PDMS matrix functionalized with spiropyran (SP) molecules, the prepolymer/curing agent weight ratio used for preparing said PDMS matrix is equal to 2.5:1, and the percentage by weight of said SP molecules in said at least one polymeric matrix is 2 wt%.

**16.** The manipulation device (1), according to any of claims 1 to 15, wherein said proximal jaw device (100) has a proximal jaw (2) comprising:

- an elastically-deformable front element (101a), which, in operation, is in contact with the tissue to be grasped (T); and
- an elastically-deformable back support element (102a), fixed to the front element (101a) and configured to receive a grasping force ($F_z$), which support element (102a) has a higher modulus of elasticity than the front element (101a) being less elastically compliant,

wherein said front element (101a) and said support element (102a) each develop according to a main longitudinal direction (x) and are fixed one another along respective longitudinal edge portions so as to define a tubular hollow structure (151, 152),

the overall arrangement being such that when the grasping force ($F_z$) is applied to said support element (102a) it deforms and transmits such force to the front element (101a) which, in turn, deforms,

both the front element (101a) and the support element (102a) deforming in a main deforming transverse direction (y) that is orthogonal to said main longitudinal direction (x) and to the grasping force ($F_z$).

**17.** A surgical apparatus for minimally-invasive surgery, comprising a manipulation device (1) according to anyone of claims 1 to 16 and an endoscope configured to show the surgeon the visual feedback from said mechanochromic indicator (3).

**18.** The surgical apparatus according to claim 17, wherein said endoscope is configured to perform an optical pumping for reverting a colour change of the mechanochromic indicator (3).

**19.** The surgical apparatus according to claim 17 or 18, comprising optical fibers arranged at, or within, pressure means (10) acting upon said mechanochromic indicator (3).

## Patentansprüche

**1.** Manipulationsvorrichtung (1) für eine minimalinvasive Operation, umfassend mindestens eine Greifvorrichtung (100), die zum Einwirken auf ein Gewebe konfiguriert ist,

wobei die Greifvorrichtung (100) einen mechanochromen Indikator (3) umfasst,

wobei der mechanochrome Indikator (3) mindestens eine Polymermatrix aufweist, die mit mechanochromen Molekülen funktionalisiert ist,

wobei der mechanochrome Indikator (3) konfiguriert ist, um den Bediener mit einer visuellen Farbrückmeldung zu versehen, wenn eine zuvor bestimmte Schwellenkraft oder ein zuvor bestimmter Schwellendruck ($-F_c$, $p_c$) auf das Gewebe ausgeübt wird,

wobei die Manipulationsvorrichtung (1) ferner einen Kraftübertragungsmechanismus (8, 5) umfasst, um eine Reaktionskraft ($F_c$), die von dem Gewebe auf eine proximale Backe (2) der Greifvorrichtung (100) ausgeübt wird, auf den mechanochromen Indikator (3) zu übertragen,

**dadurch gekennzeichnet, dass** die Manipulationsvorrichtung (1) ferner ein Druckmittel (10) umfasst, das konfiguriert ist, um einen Druck auf den mechanochromen Indikator (3) auszuüben,

wobei der Kraftübertragungsmechanismus konfiguriert ist, so dass das Druckmittel (10) einen Druck auf den mechanochromen Indikator (3) ausübt, der in Bezug auf den Druck, der von dem Gewebe auf die proximale Backe (2) ausgeübt wird, verstärkt ist.

**2.** Manipulationsvorrichtung (1) nach Anspruch 1, wobei der Kraftübertragungsmechanismus ein Mechanismus einer Hebelart ist, vorzugsweise ein Hebelmechanismus einer ersten Art, der einem zentralen Drehpunkt (8) aufweist.

**3.** Manipulationsvorrichtung (1) nach Anspruch 2, wobei der Mechanismus der Hebelart eine Schwenkachse ($P_y$) aufweist, die sich gemäß einer Schwenkrichtung (y) schräg zu sowohl einer Gewebekraftausübungsrichtung (z) als auch einer längsgängigen Richtung einer Entwicklung (x) der proximalen Backenvorrichtung (100) erstreckt.

**4.** Manipulationsvorrichtung (1) nach einem der Ansprüche 1 bis 3, wobei das Druckmittel (10) einen Druck auf einen ausgewählten Bereich des mechanochromen Indikators (3) ausübt.

**5.** Manipulationsvorrichtung (1) nach einem der Ansprüche 1 bis 4, wobei das Druckmittel ein oder mehrere Eindringkörperelemente (10) umfasst, die von einem Basiselement (5) hervorstehen, wobei das eine oder die mehreren Eindringkörperelemente (10) jeweils vorzugsweise als eine Platte oder eine Säule konfiguriert sind.

**6.** Manipulationsvorrichtung (1) nach einem der Ansprüche 1 bis 5, wobei das Druckmittel (110) konfiguriert ist, um eine visuelle Nachricht, insbesondere eine alphanumerische Nachricht, auf den mechanochromen Indikator (3) aufzudrucken.

**7.** Manipulationsvorrichtung (1) nach einem der Ansprüche 1 bis 6, wobei der Druck $p_i$, der auf den mechanochromen Indikator (3) ausgeübt wird, und der Druck $p_c$, der auf die proximale Backe (2) ausgeübt wird, durch die folgende Gleichung verknüpft sind:

$$p_i \cong p_c\ (A_c/A_i),$$

wobei $A_c$ und Ai die Druckausübungsfläche auf die proximale Backe (2) beziehungsweise den mechanochromen Indikator (3) bezeichnen.

**8.** Manipulationsvorrichtung (1) nach einem der Ansprüche 1 bis 7, umfassend ein oder mehrere transparente Elemente (5, 6, 10), um dem Bediener zu ermöglichen, eine visuelle Rückmeldung von dem mechanochromen Indikator (3) aufzunehmen.

**9.** Manipulationsvorrichtung (1) nach einem der Ansprüche 1 bis 8, wobei der mechanochrome Indikator (3) im Wesentlichen als ein Ziegel geformt ist.

**10.** Manipulationsvorrichtung (1) nach einem der Ansprüche 1 bis 9, wobei der mechanochrome Indikator (3) einen reversiblen Farbwechsel aufweist.

**11.** Manipulationsvorrichtung (1) nach einem der Ansprüche 1 bis 10, wobei der mechanochrome Indikator (3) mindestens eine Polymermatrix umfasst, die mit mechanochromen Molekülen funktionalisiert ist, **dadurch gekennzeichnet, dass:**

- ein Präpolymer/Härtemittel-Gewichtsverhältnis, das zum Herstellen der mindestens einen Polymermatrix verwendet wird, in einem Bereich von etwa 1 : 1-15:1 vorliegt; und
- ein Gewichtsprozentsatz der mechanochromen Moleküle in der mindestens einen Matrix in einem Bereich von etwa 0,1-5 % vorliegt.

**12.** Manipulationsvorrichtung (1) nach Anspruch 11, wobei die mindestens eine Polymermatrix aus der Gruppe ausgewählt ist, umfassend Polydimethylsiloxan (PDMS), Poly(methylacrylat) (PMA), Polycaprolacton (PCL), Polymethylmethacrylat (PMMA), Polystyrol und Poly(n-butylacrylat)-triblockcopolymere ((PS)-PnBA-SP-PnBA-PS),), Ureidopyrimidinon (UPy) und/oder Kombinationen davon.

**13.** Manipulationsvorrichtung (1) nach Anspruch 11 oder 12, wobei die mechanochromen Moleküle funktionalisierte Spiropyran-Moleküle (SP-Moleküle) sind.

**14.** Manipulationsvorrichtung (1) nach einem der Ansprüche 11 bis 13, wobei die mechanochromen Moleküle an die mindestens eine Polymermatrix kovalent gebunden sind.

**15.** Manipulationsvorrichtung (1) nach einem der Ansprüche 11 bis 14, wobei die mindestens eine Polymermatrix eine PDMS-Matrix ist, die mit Spiropyran-Molekülen (SP-Molekülen) funktionalisiert ist, das Präpolymer/Härtungsmittel-Gewichtsverhältnis, das zum Herstellen der PDMS-Matrix verwendet wird, gleich 2,5 : 1 ist und der Gewichtsprozentsatz der SP-Moleküle in der mindestens einen Polymermatrix 2 Gew.-% beträgt.

**16.** Manipulationsvorrichtung (1) nach einem der Ansprüche 1 bis 15, wobei die proximale Backenvorrichtung (100) eine proximale Backe (2) aufweist, umfassend:

- ein elastisch verformbares vorderes Element (101a), das, im Betrieb, mit dem zu greifenden (T) Gewebe in Kontakt steht; und
- ein elastisch verformbares hinteres Stützelement (102a), das an dem vorderen Element (101a) befestigt und konfiguriert ist, um eine Greifkraft ($F_Z$) aufzunehmen, wobei das Stützelement (102a) einen höheren Elastizitätsmodul als das vordere Element (101a) aufweist und weniger elastisch nachgiebig ist,

wobei sich das vordere Element (101a) und das Stützelement (102a) jeweils gemäß einer Hauptlängsrichtung (x) entwickeln und entlang jeweiliger Längskantenabschnitte aneinander befestigt sind, um eine röhrenförmige Hohlstruktur (151, 152) zu definieren,
wobei die Gesamtanordnung derart ist, dass, wenn die Greifkraft ($F_z$) auf das Stützelement (102a) ausgeübt wird, es sich verformt und eine derartige Kraft auf das vordere Element (101a) überträgt, das sich wiederum verformt,
wobei sich sowohl das vordere Element (101a) als auch das Stützelement (102a) in einer Hauptverformungsquerrichtung (y) verformen, die orthogonal zu der Hauptlängsrichtung (x) und zu der Greifkraft ($F_z$) ist.

**17.** Chirurgische Einrichtung für die minimalinvasive Operation, umfassend eine Manipulationsvorrichtung (1) nach einem der Ansprüche 1 bis 16 und ein Endoskop, das konfiguriert ist, um dem Chirurgen die visuelle Rückmeldung von dem mechanochromen Indikator (3) anzuzeigen.

**18.** Chirurgische Einrichtung nach Anspruch 17, wobei das Endoskop konfiguriert ist, um ein optisches Pumpen zum Umkehren einer Farbänderung des mechanochromen Indikators (3) durchzuführen.

**19.** Chirurgische Einrichtung nach Anspruch 17 oder 18, umfassend optische Fasern, die an, oder innerhalb, des Druckmittels (10) angeordnet sind, die auf den mechanochromen Indikator (3) einwirken.

**Revendications**

**1.** Dispositif de manipulation (1) pour une chirurgie mini-invasive, comprenant au moins un dispositif de préhension (100) conçu pour agir sur un tissu,

lequel dispositif de préhension (100) comprend un indicateur mécanochromique (3),
dans lequel ledit indicateur mécanochromique (3) a au moins une matrice polymère fonctionnalisée avec des molécules mécanochromiques,
dans lequel ledit indicateur mécanochromique (3) est configuré pour fournir à l'opérateur un retour visuel en couleur lorsqu'une force ou une pression seuil prédéterminée (- $F_c$, $p_c$) est appliquée sur le tissu,
dans lequel ledit dispositif de manipulation (1) comprend en outre un mécanisme de transmission de force (8, 5) pour transférer une force de réaction ($F_c$) appliquée par le tissu sur une mâchoire proximale (2) dudit dispositif de préhension (100) audit indicateur mécanochromique (3),
**caractérisé en ce que** ledit dispositif de manipulation (1) comprend en outre un moyen de pression (10) conçu pour appliquer une pression sur ledit indicateur mécanochromique (3),
dans lequel ledit mécanisme de transmission de force est conçu de telle sorte que ledit moyen de pression (10) applique une pression sur ledit indicateur mécanochromique (3) qui est amplifiée par rapport à la pression appliquée par le tissu sur ladite mâchoire proximale (2).

**2.** Dispositif de manipulation (1) selon la revendication 1, dans lequel ledit mécanisme de transmission de force est un mécanisme de type levier, de préférence un mécanisme à levier de premier type ayant un point d'appui central (8).

**3.** Dispositif de manipulation (1) selon la revendication 2, dans lequel ledit mécanisme de type levier a un axe de pivotement ($P_y$) s'étendant selon une direction de pivotement (y) traversant à la fois une direction d'application de force tissulaire(z) et une direction de développement prévalente longitudinale (x) dudit dispositif de mâchoire proximale (100).

**4.** Dispositif de manipulation (1) selon l'une quelconque des revendications 1 à 3, dans lequel ledit moyen de pression (10) applique une pression sur une zone sélectionnée dudit indicateur mécanochromique (3).

**5.** Dispositif de manipulation (1) selon l'une quelconque des revendications 1 à 4, dans lequel ledit moyen de pression comprend un ou plusieurs éléments pénétrateurs (10) faisant saillie à partir d'un élément de base (5), ledit ou lesdits éléments de pénétration (10) étant chacun de préférence conçu comme une plaque ou un pilier.

**6.** Dispositif de manipulation (1) selon l'une quelconque des revendications 1 à 5, dans lequel ledit moyen de pression (110) est conçu pour imprimer un message visuel, en particulier un message alphanumérique, sur ledit indicateur mécanochromique (3).

**7.** Dispositif de manipulation (1) selon l'une quelconque des revendications 1 à 6, dans lequel la pression $p_i$ appliquée sur ledit indicateur mécanochromique (3) et la pression $p_c$ appliquée sur ladite mâchoire proximale (2) sont liées par l'équation suivante :

$$p_i \cong p_c(A_c/A_i),$$

où $A_c$ et $A_i$ désignent la zone d'application de pression sur la mâchoire proximale (2) et l'indicateur mécanochromique (3), respectivement.

**8.** Dispositif de manipulation (1) selon l'une quelconque des revendications 1 à 7, comprenant un ou plusieurs éléments transparents (5, 6, 10) pour permettre à l'opérateur de recevoir un retour visuel à partir dudit indicateur mécanochromique (3).

**9.** Dispositif de manipulation (1) selon l'une quelconque des revendications 1 à 8, dans lequel l'indicateur mécanochromique (3) a sensiblement la forme d'une brique.

**10.** Dispositif de manipulation (1) selon l'une quelconque des revendications 1 à 9, dans lequel l'indicateur mécanochromique (3) a un changement de couleur réversible.

**11.** Dispositif de manipulation (1) selon l'une quelconque des revendications 1 à 10, dans lequel ledit indicateur mécanochromique (3) comprend au moins une matrice polymère fonctionnalisée avec des molécules mécanochromiques, **caractérisé en ce que** :

- un rapport de poids prépolymère/agent de durcissement utilisé pour préparer ladite au moins une matrice polymère est compris dans une plage d'environ 1:1 à 15:1 ; et
- un pourcentage en poids desdites molécules mécanochromiques dans ladite au moins une matrice est compris dans une plage d'environ 0,1 à 5 %.

**12.** Dispositif de manipulation (1), selon la revendication 11, dans lequel ladite au moins une matrice polymère est choisie dans le groupe comprenant polydiméthylsiloxane (PDMS), poly(méthylacrilate) (PMA), polycaprolactone (PCL), polyméthacrylate de méthyle (PMMA), copolymères triblocs de polystyrène et de poly(n-butyl acrylate) ((PS)-PnBA-SP-PnBA-PS),), uréidopyrimidinone (UPy) et/ou leurs combinaisons.

**13.** Dispositif de manipulation (1), selon les revendications 11 ou 12, dans lequel lesdites molécules mécanochromiques sont des molécules de spiropyrane (SP) fonctionnalisées.

**14.** Dispositif de manipulation (1) selon l'une quelconque des revendications 11 à 13, dans lequel lesdites molécules mécanochromiques sont liées de manière covalente à ladite au moins une matrice polymère.

**15.** Dispositif de manipulation (1) selon l'une quelconque des revendications 11 à 14, dans lequel ladite au moins une matrice polymère est une matrice PDMS fonctionnalisée avec des molécules de spiropyranne (SP), le rapport de poids prépolymère/agent de durcissement utilisé pour préparer ladite matrice PDMS est égal à 2,5:1, et le pourcentage en poids desdites molécules SP dans ladite au moins une matrice polymère est de 2 % en poids.

**16.** Dispositif de manipulation (1) selon l'une quelconque des revendications 1 à 15, dans lequel ledit dispositif de mâchoire proximale (100) a une mâchoire proximale (2) comprenant :

- un élément frontal (101a) élastiquement déformable qui, lors du fonctionnement, est en contact avec le tissu à saisir (T) ; et
- un élément de support arrière (102a) élastiquement déformable, fixé à l'élément frontal (101a) et conçu pour recevoir une force de préhension ($F_z$), lequel élément de support (102a) a un module d'élasticité plus élevé que l'élément frontal (101a) étant moins élastiquement conforme,

dans lequel ledit élément frontal (101a) et ledit élément de support (102a) se développent chacun selon une direction longitudinale principale (x) et sont fixés l'un à l'autre le long des parties de bord longitudinal respectives de manière à définir une structure creuse tubulaire (151, 152),
l'agencement global étant tel que lorsque la force de préhension ($F_z$) est appliquée audit élément de support

(102a), il se déforme et transmet une telle force à l'élément frontal (101a) qui, à son tour, se déforme, à la fois l'élément frontal (101a) et l'élément de support (102a) se déforment dans une direction transversale principale de déformation (y) qui est orthogonale à ladite direction longitudinale principale *(x)* et à la force de préhension ($F_z$).

**17.** Appareil chirurgical pour une chirurgie mini-invasive, comprenant un dispositif de manipulation (1) selon l'une quelconque des revendications 1 à 16 et un endoscope conçu pour montrer au chirurgien le retour visuel dudit indicateur mécanochromique (3).

**18.** Appareil chirurgical selon la revendication 17, dans lequel ledit endoscope est conçu pour effectuer un pompage optique afin d'inverser un changement de couleur de l'indicateur mécanochromique (3).

**19.** Appareil chirurgical selon la revendication 17 ou 18, comprenant des fibres optiques disposées au niveau ou à l'intérieur du moyen de pression (10) agissant sur ledit indicateur mécanochromique (3).

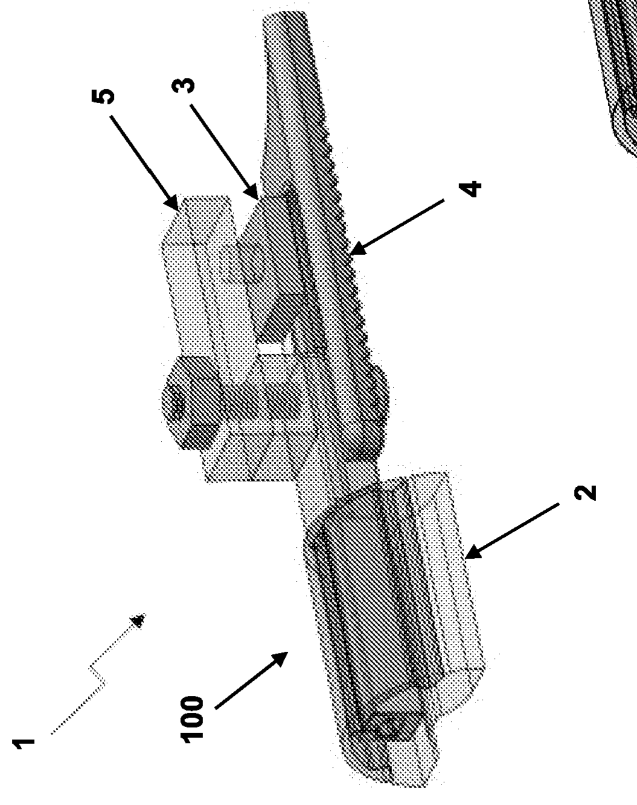


FIG. 1A

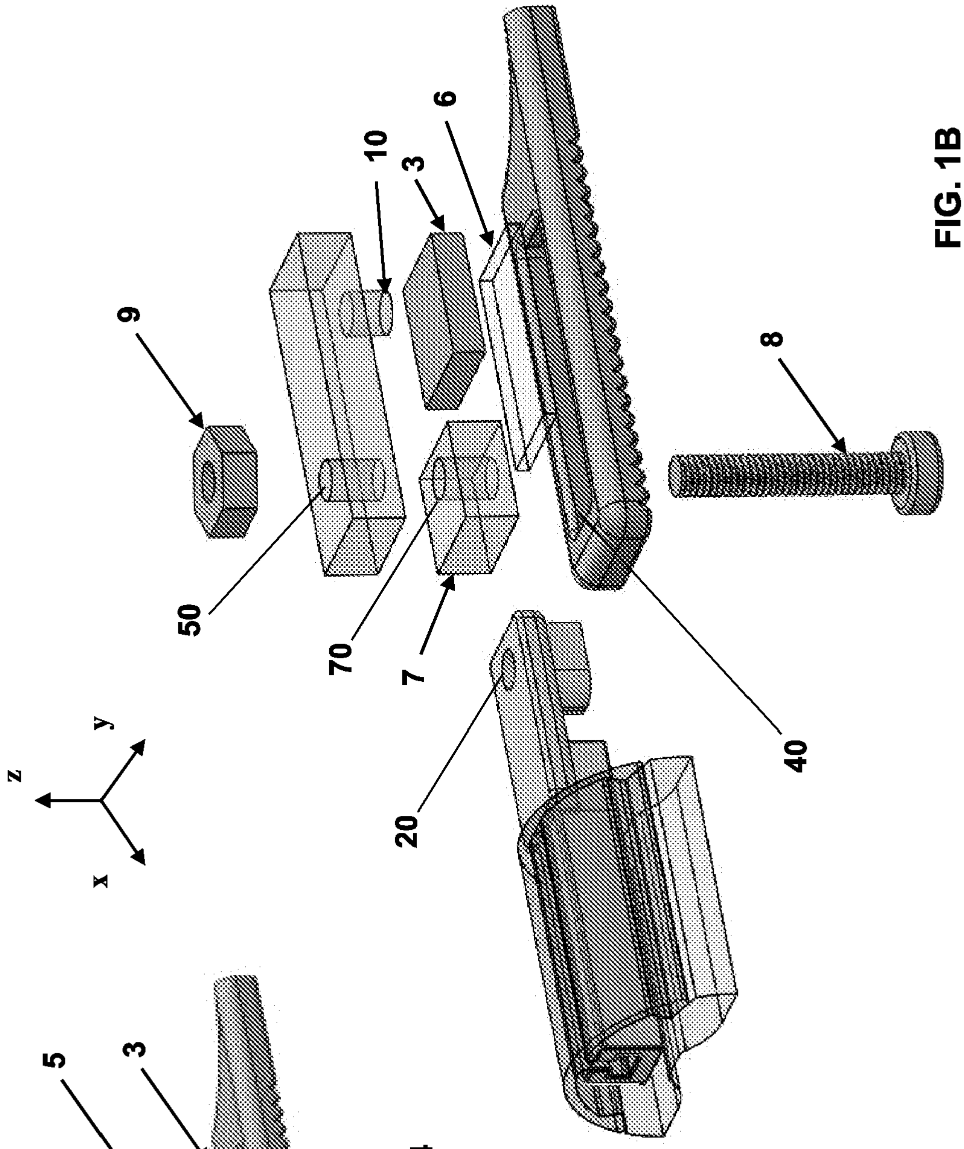


FIG. 1B

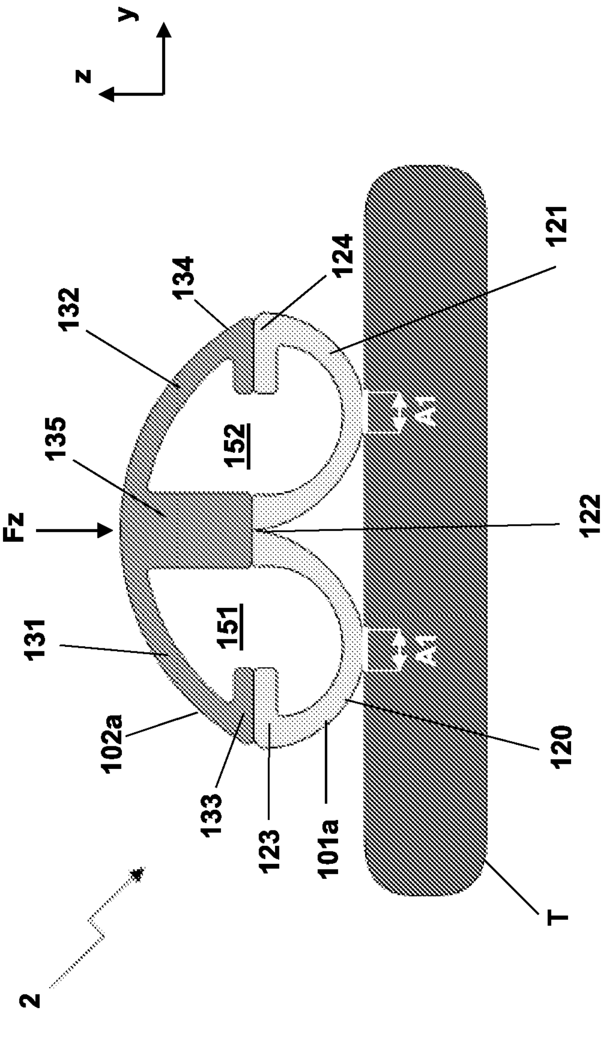
2
Fz
z
y
131
135
132
134
124
121
102a
151
152
122
133
123
101a
120
A1
A1
T

FIG. 1C

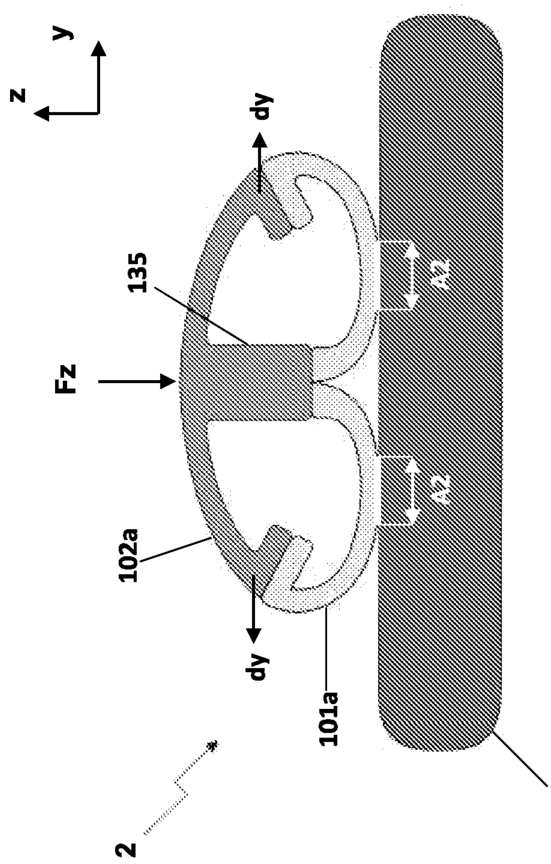
2
z
y
Fz
135
dy
dy
102a
101a
A2
A2
T

FIG. 1D

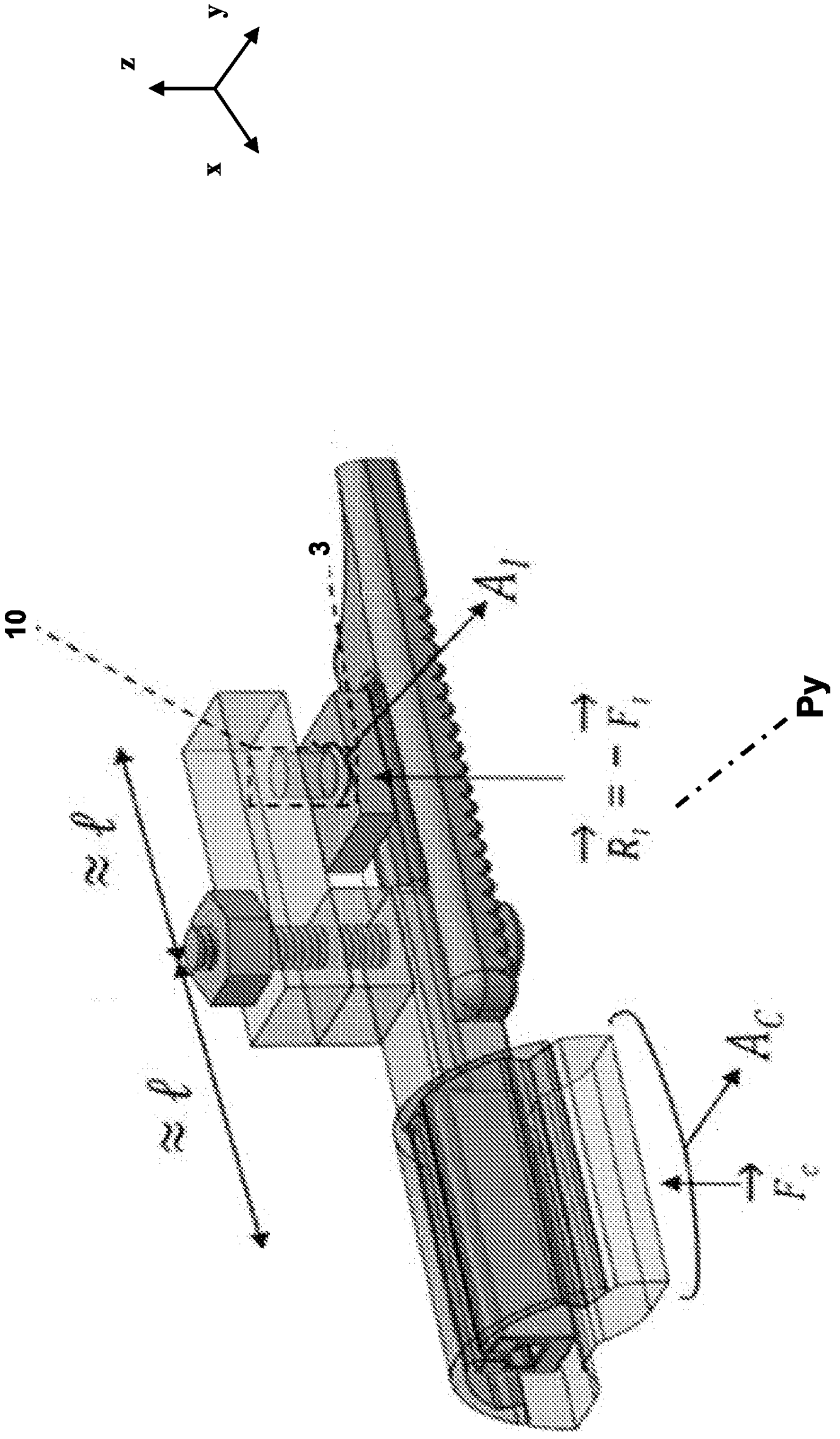


FIG. 1E

FIG. 2A

FIG. 2B

FIG. 2C

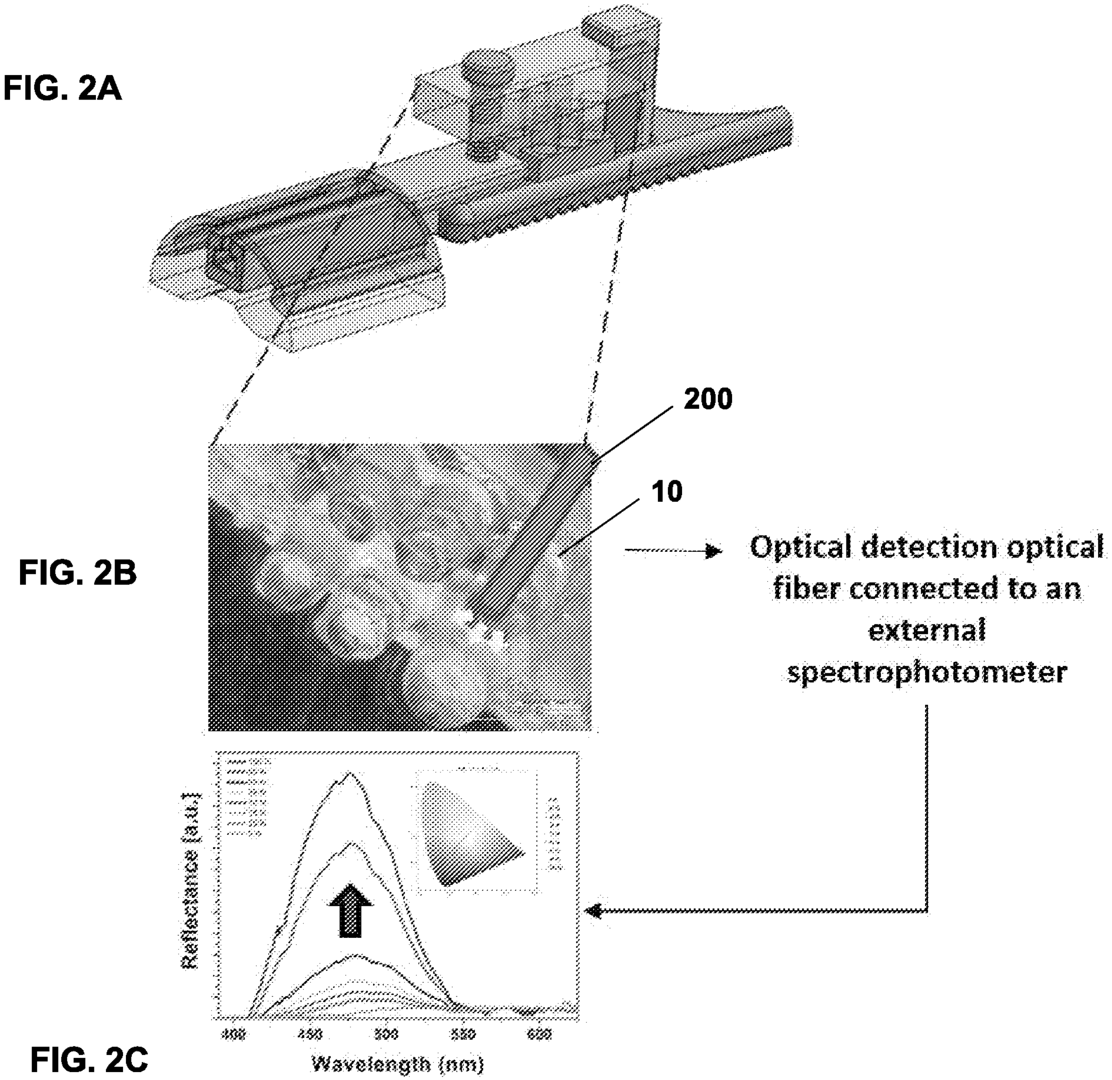


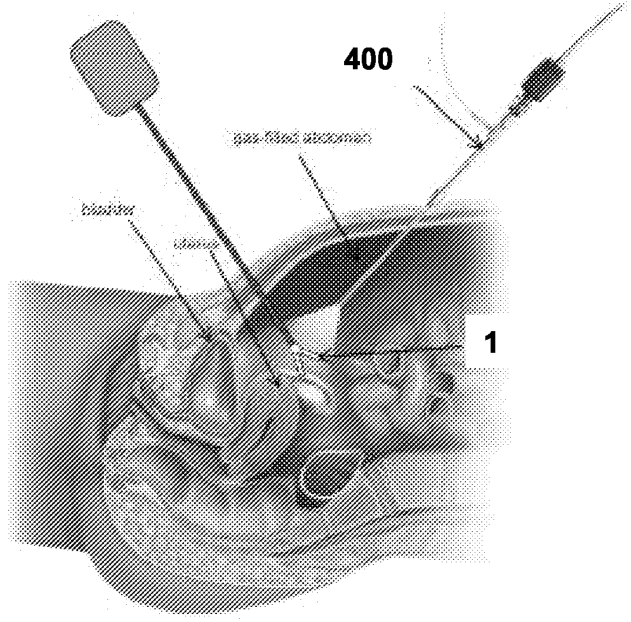


FIG. 3

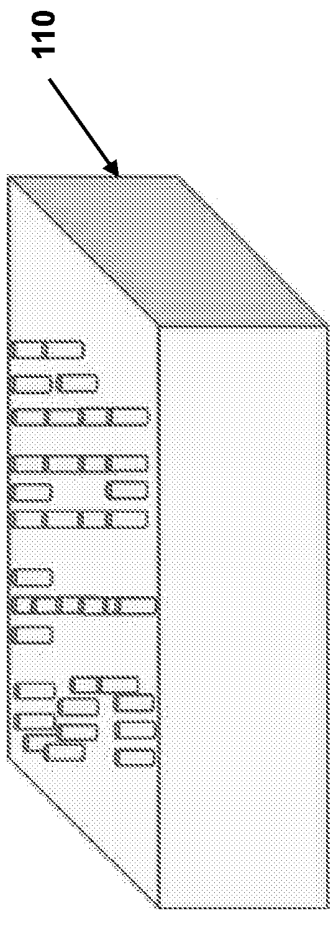


FIG. 4

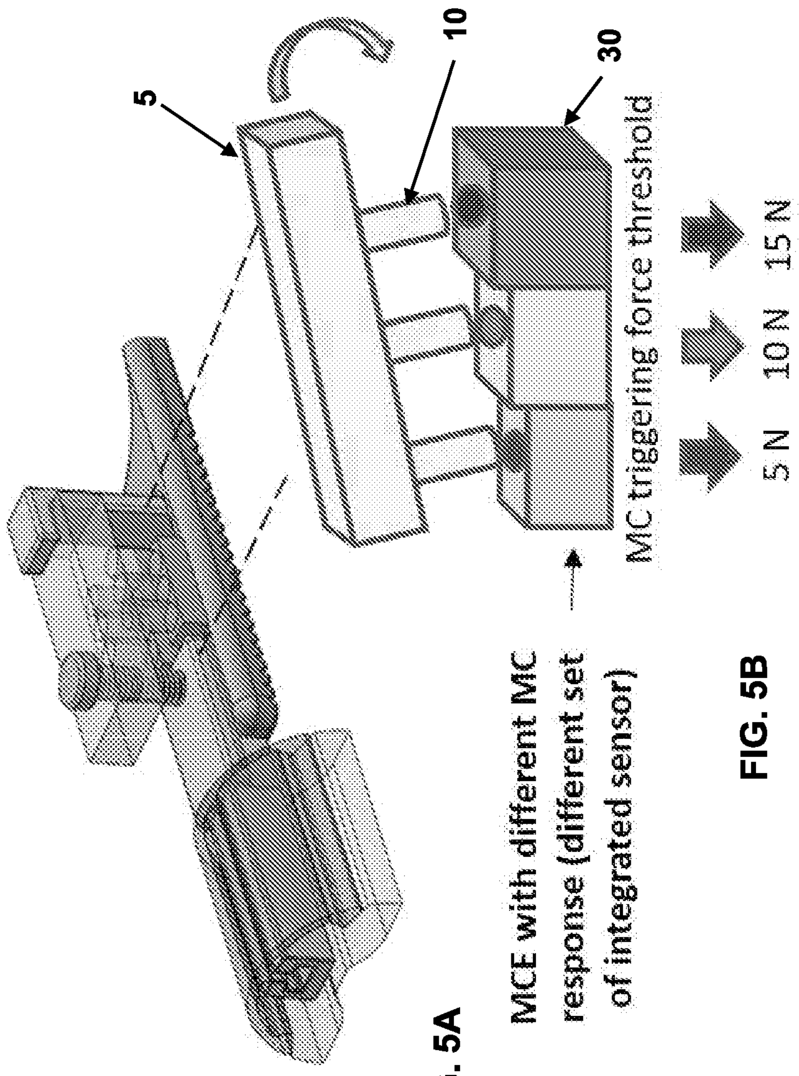


FIG. 5A

FIG. 5B

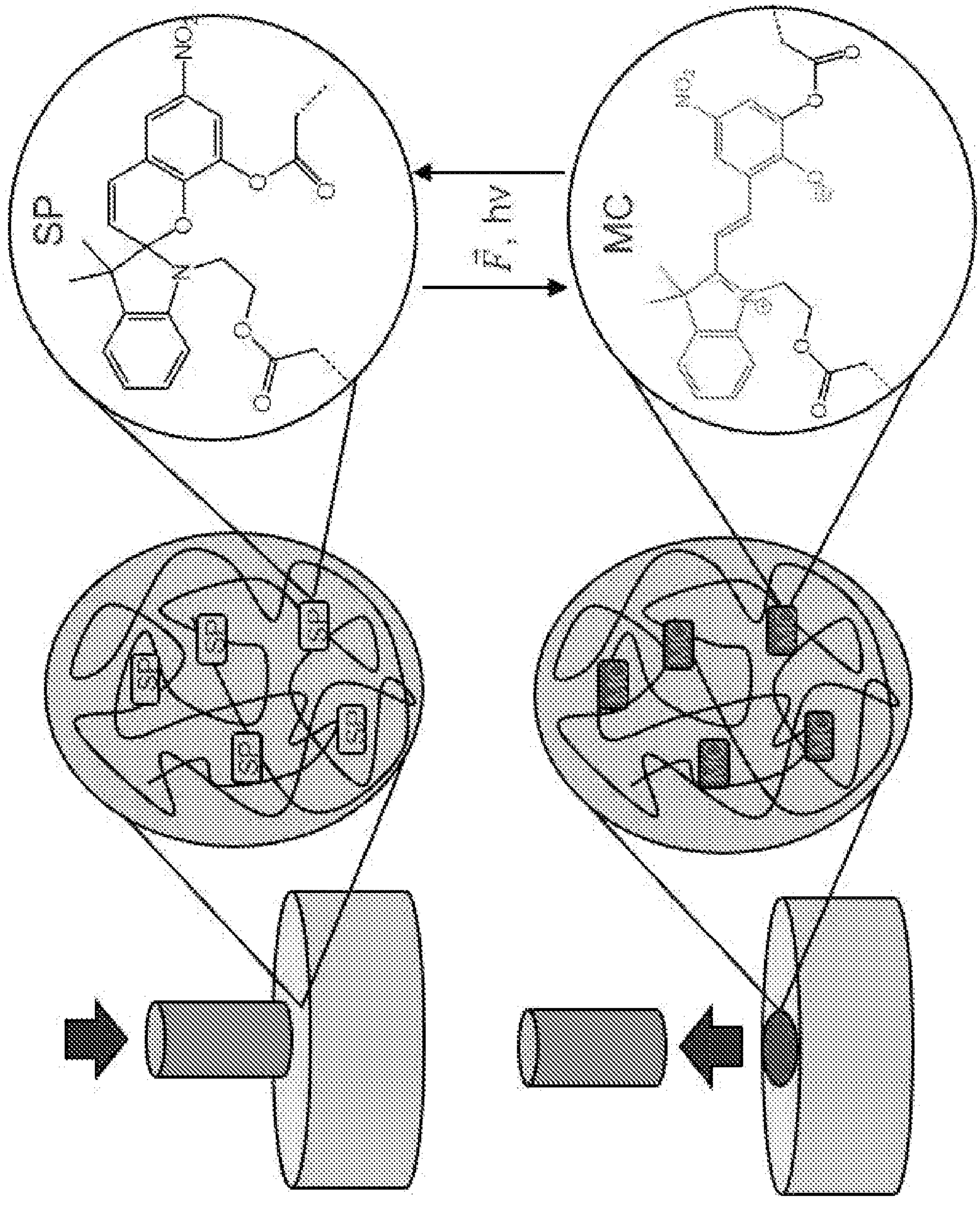


FIG. 6

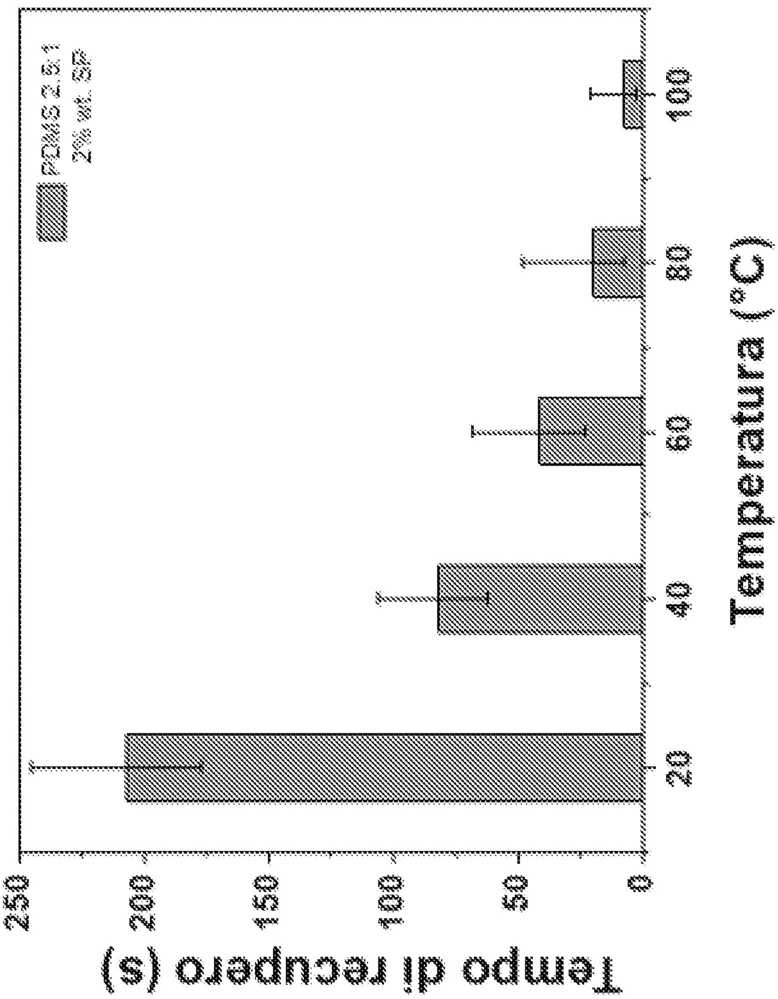

Tempo di recupero (s)
250
200
150
100
50
0
20
40
60
80
100
Temperatura (°C)
PDMS 2.5:1
2% wt. SP


FIG. 7

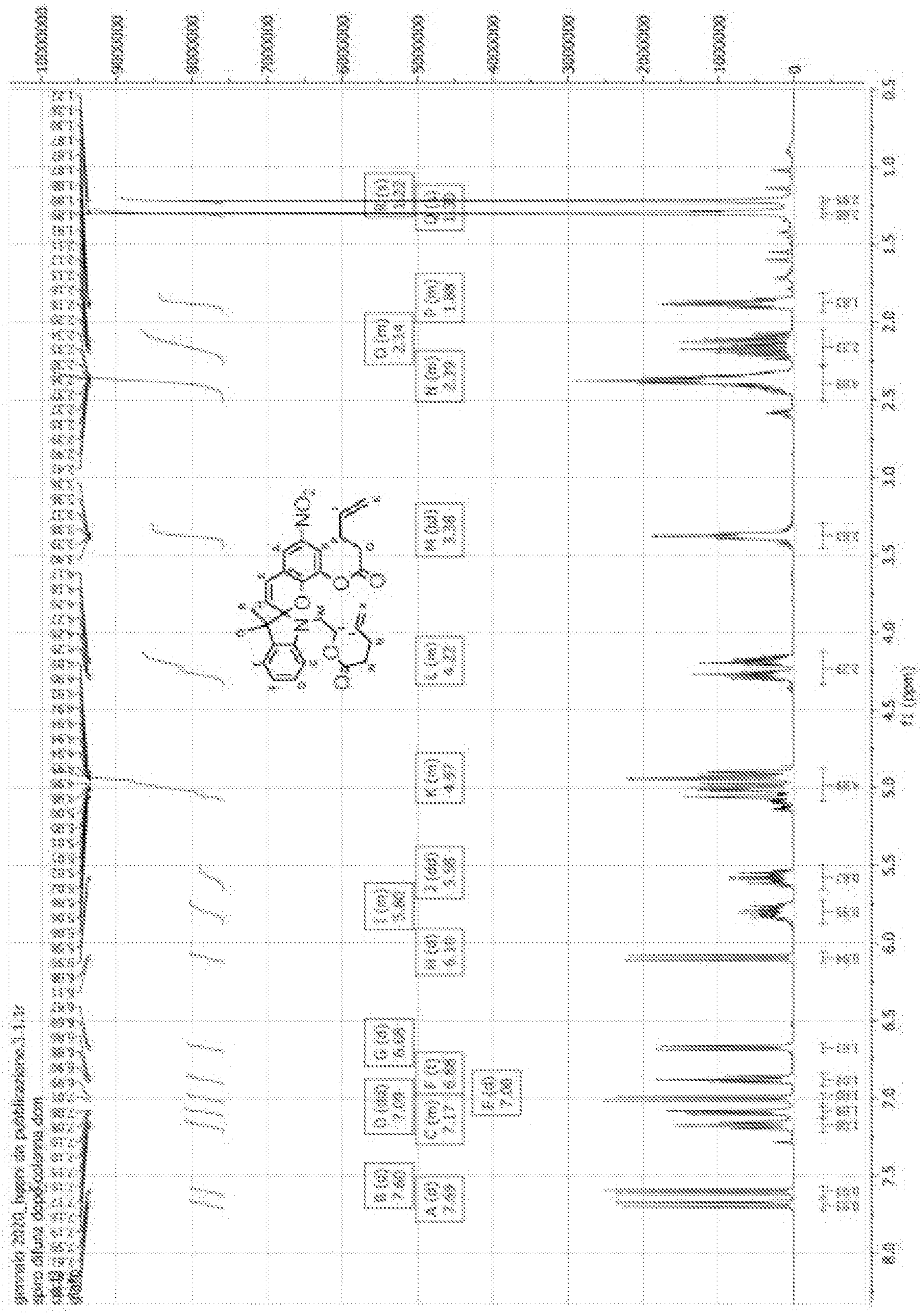

FIG. 8

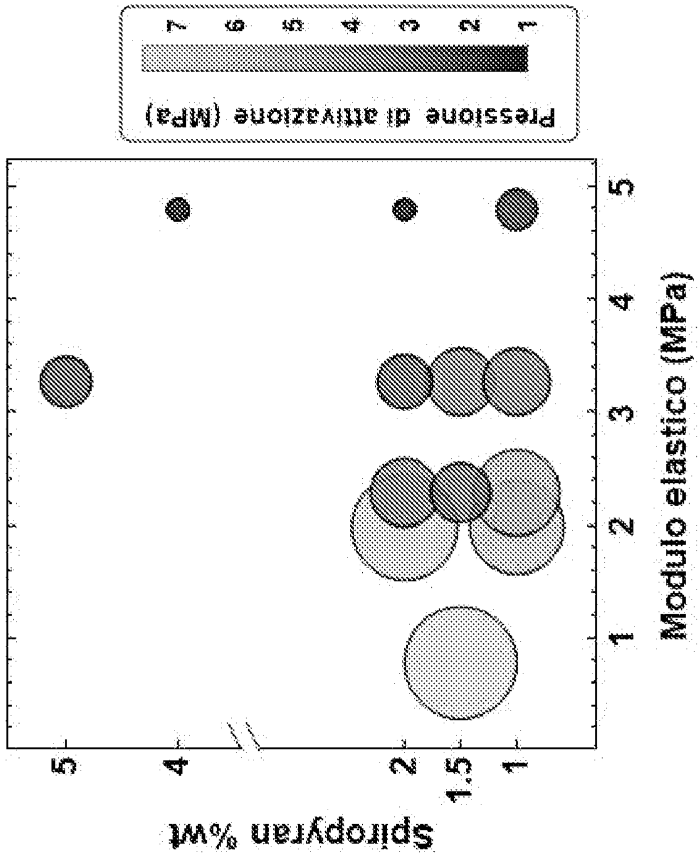
Spiropyran %wt
5
4
2
1.5
1
Modulo elastico (MPa)
1
2
3
4
5
Pressione di attivazione (MPa)
7
6
5
4
3
2
1

FIG. 9A

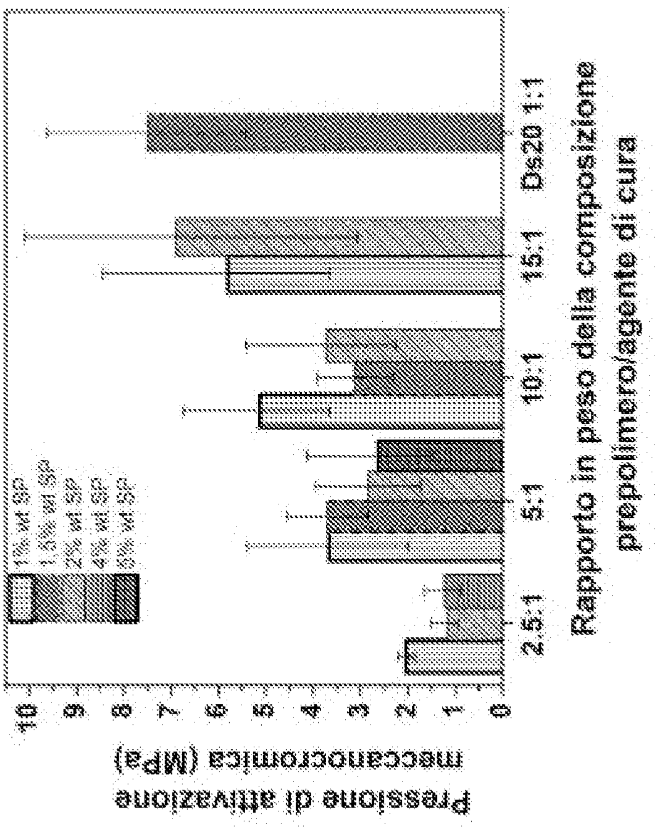
Pressione di attivazione meccanocromica (MPa)
0
1
2
3
4
5
6
7
8
9
10
1% wt SP
1.5% wt SP
2% wt SP
4% wt SP
5% wt SP
2.5:1
5:1
10:1
15:1
Ds20 1:1
Rapporto in peso della composizione prepolimero/agente di cura

FIG. 9B

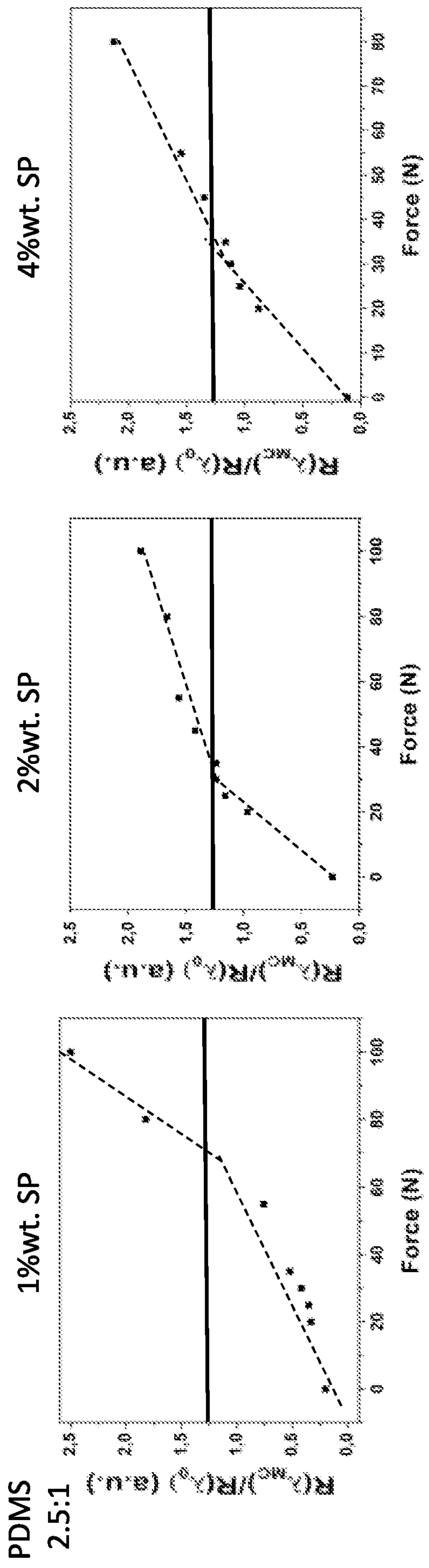


FIG. 10A

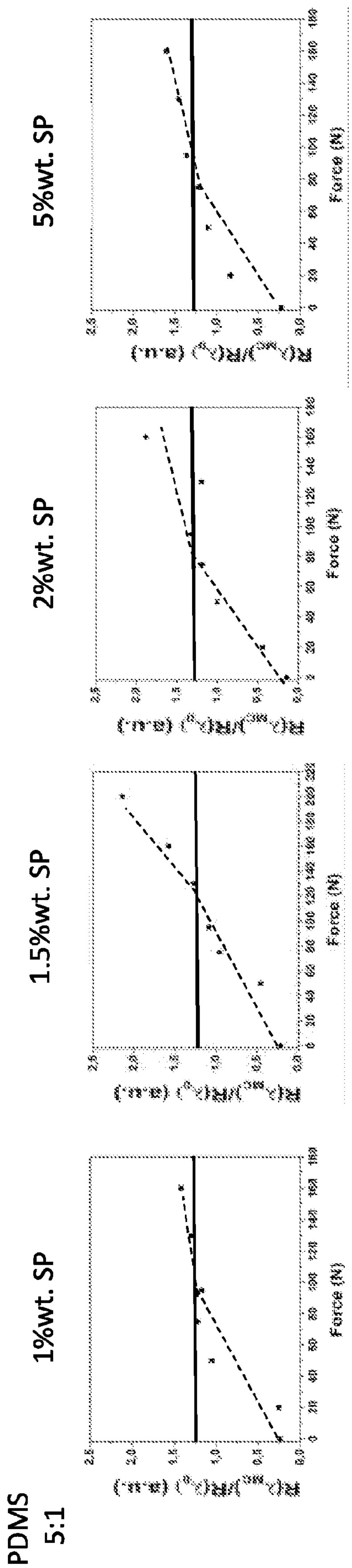


FIG. 10B

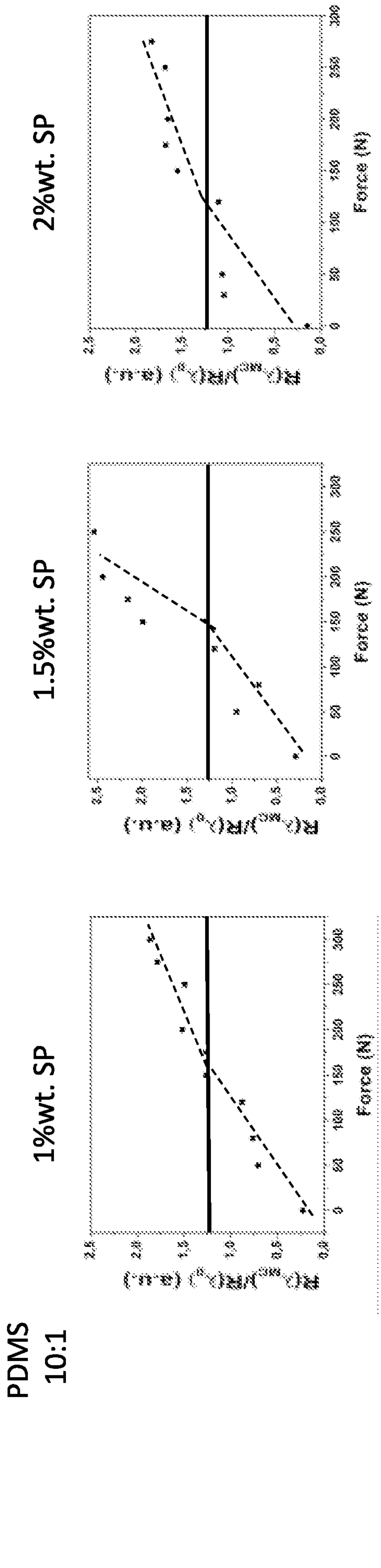


FIG. 10C

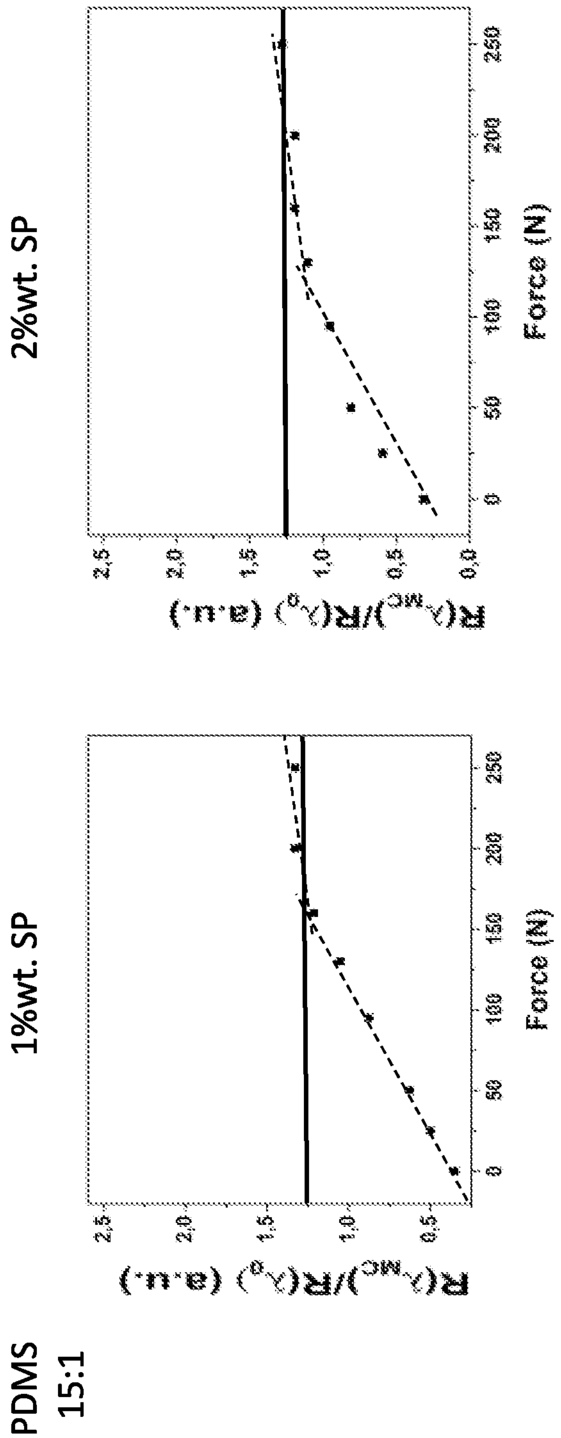
PDMS
15:1
1%wt. SP
2%wt. SP
R(λMC)/R(λo) (a.u.)
2,5
2,0
1,5
1,0
0,5
0,0
0
50
100
150
200
250
Force (N)

FIG. 10D

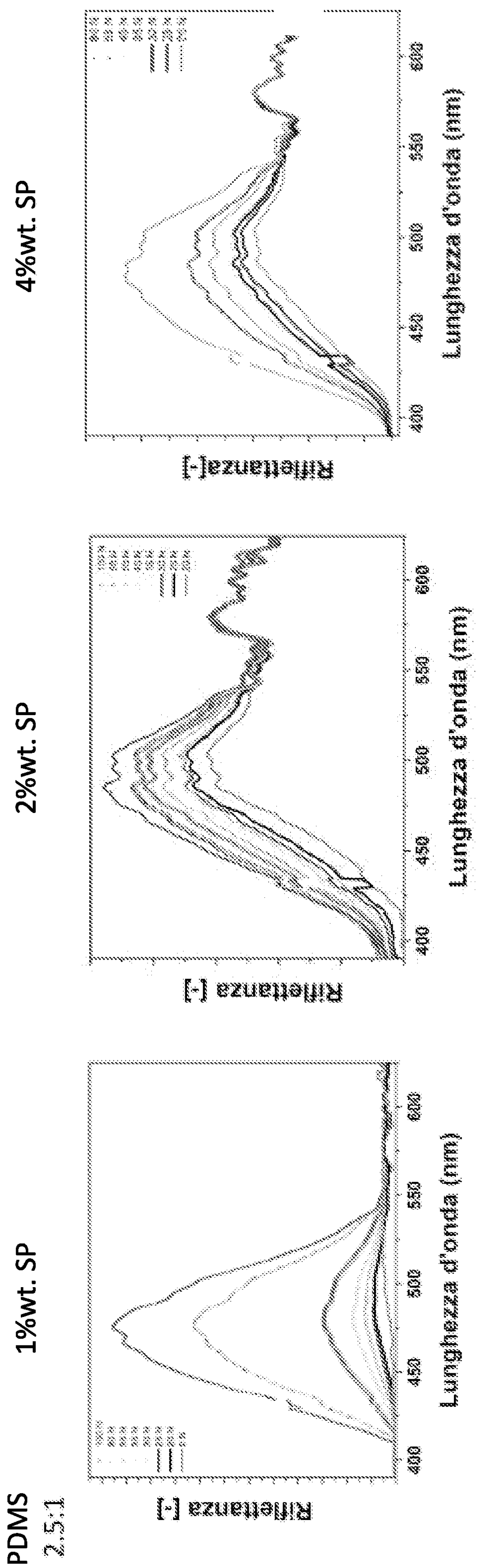
PDMS
2.5:1
1%wt. SP
2%wt. SP
4%wt. SP
Riflettanza [-]
Riflettanza [-]
Riflettanza[-]
Lunghezza d'onda (nm)
Lunghezza d'onda (nm)
Lunghezza d'onda (nm)
400
450
500
550
600

FIG. 11A

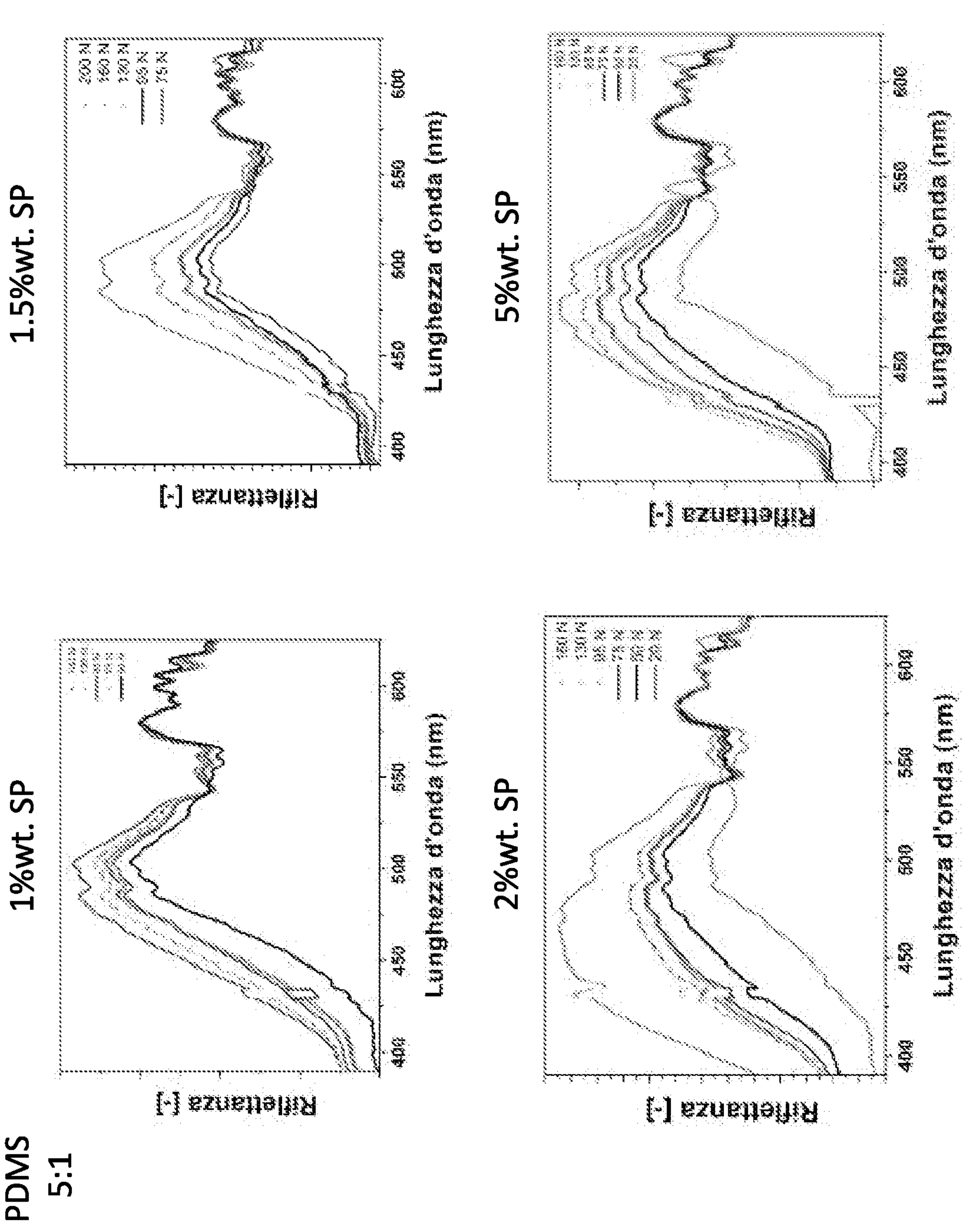
PDMS
5:1
1%wt. SP
1.5%wt. SP
2%wt. SP
5%wt. SP
Riflettanza [-]
Lunghezza d'onda (nm)
400
450
500
550
600

FIG. 11B

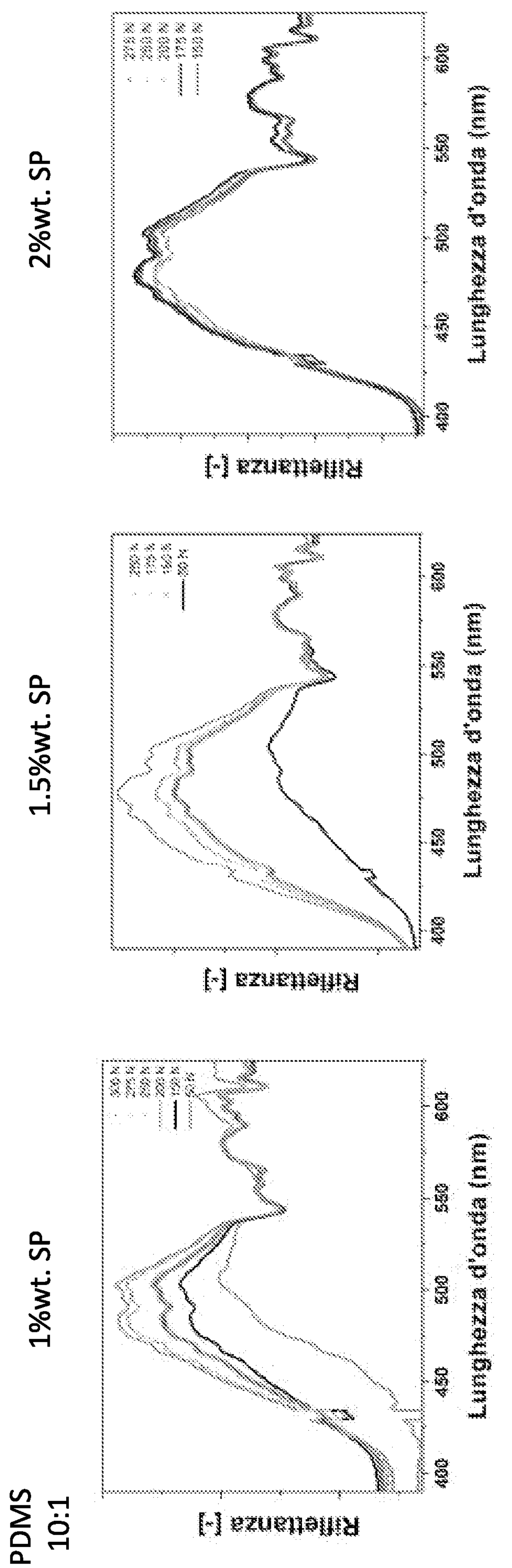
PDMS
10:1
1%wt. SP
1.5%wt. SP
2%wt. SP
Riflettanza [-]
Riflettanza [-]
Riflettanza [-]
400
450
500
550
600
Lunghezza d'onda (nm)
Lunghezza d'onda (nm)
Lunghezza d'onda (nm)

FIG. 11C

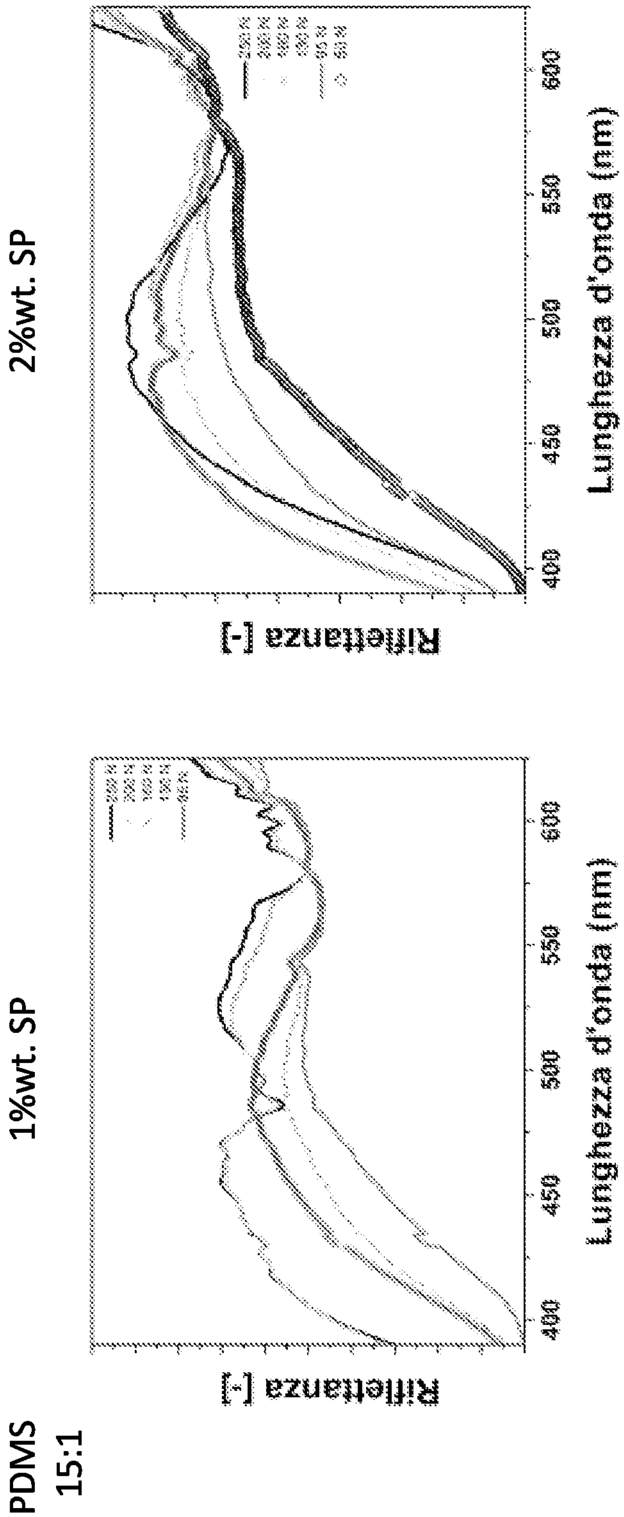
PDMS
15:1
1%wt. SP
2%wt. SP
Riflettanza [-]
Lunghezza d'onda (nm)
400
450
500
550
600

FIG. 11D

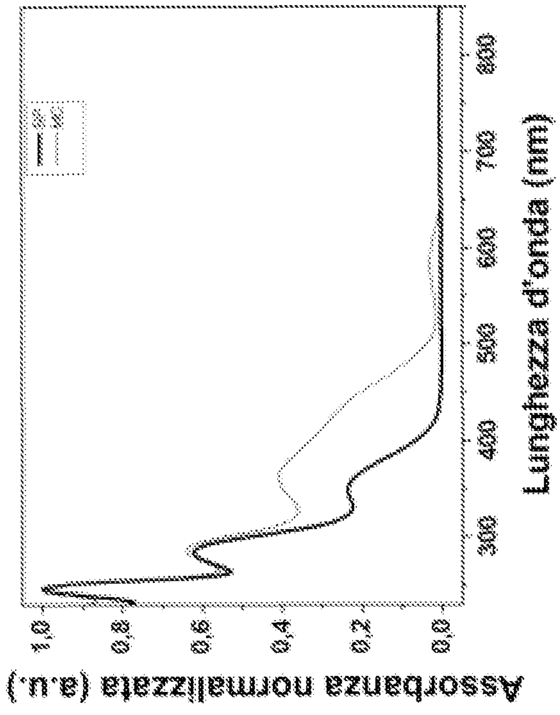
Assorbanza normalizzata (a.u.)
Lunghezza d'onda (nm)
1,0
0,8
0,6
0,4
0,2
0,0
300
400
500
600
700
800

FIG. 12

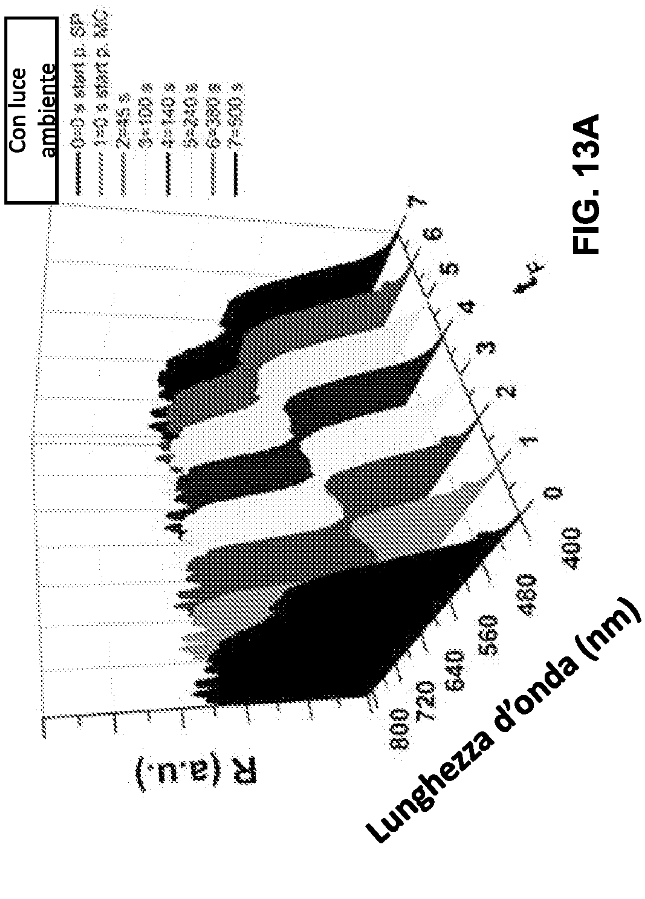


FIG. 13A

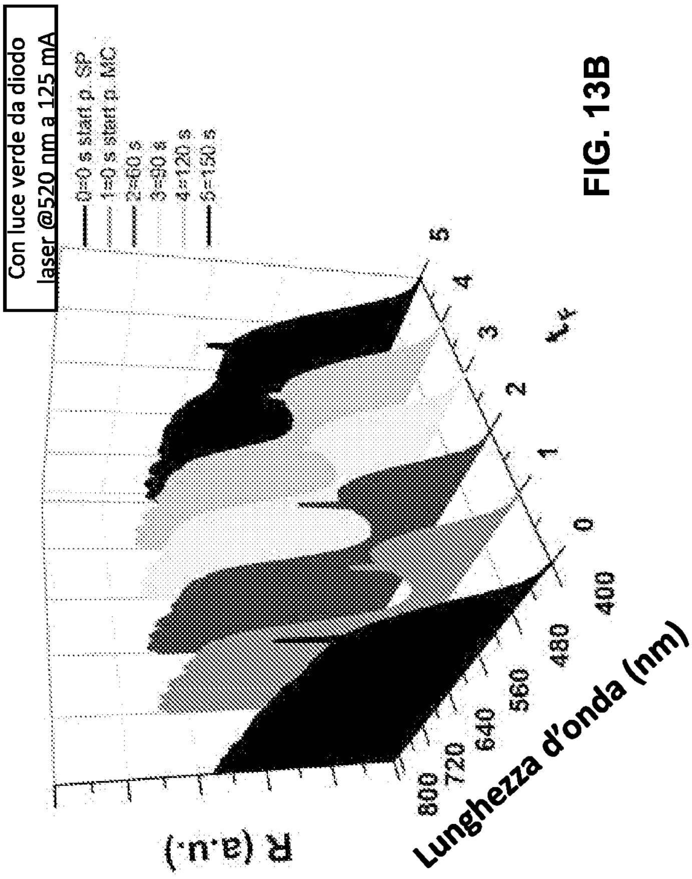


FIG. 13B

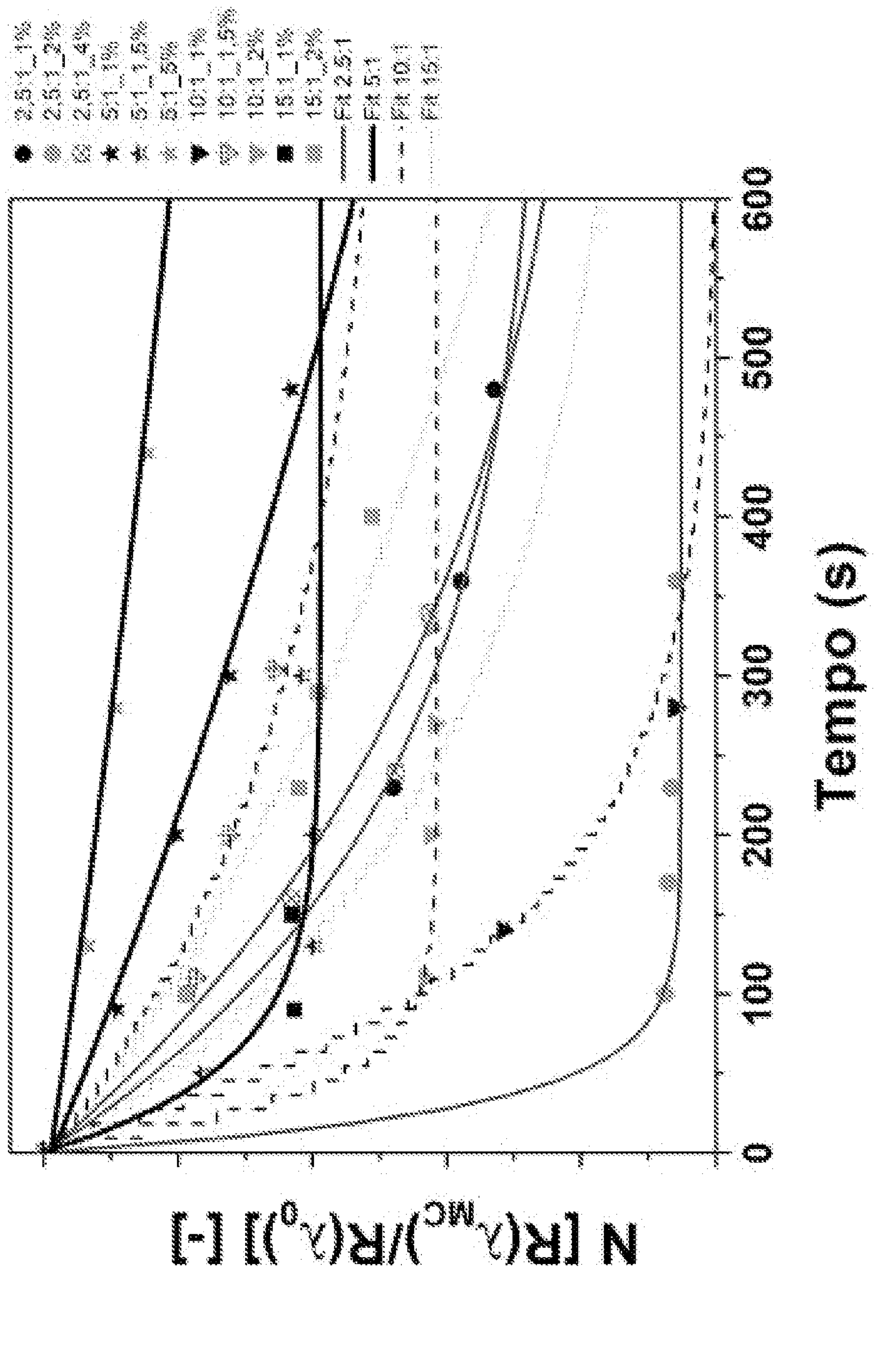
N [R(λMC)/R(λ0)] [-]
Tempo (s)
0
100
200
300
400
500
600
2.5:1_1%
2.5:1_3%
2.5:1_4%
5:1_1%
5:1_1.5%
5:1_5%
10:1_1%
10:1_1.5%
10:1_2%
15:1_1%
15:1_2%
Fit 2.5:1
Fit 5:1
Fit 10:1
Fit 15:1

FIG. 13C

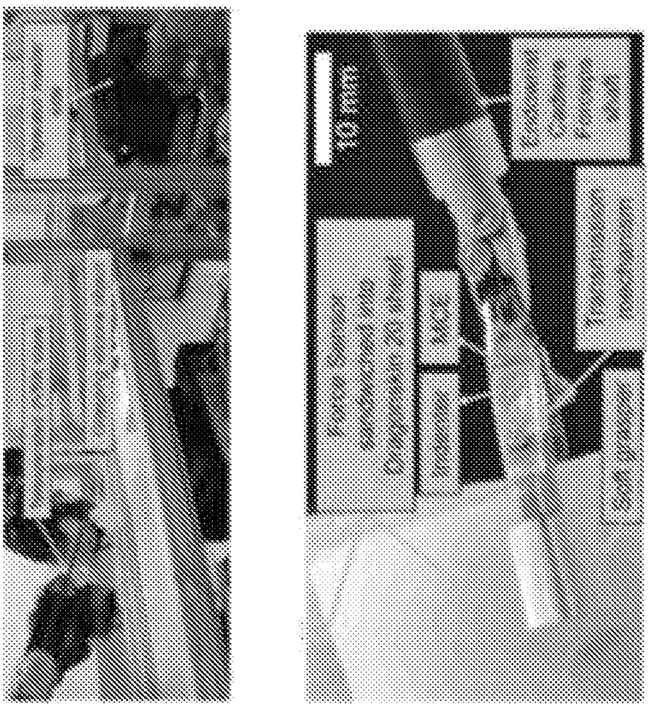


FIG. 14 A

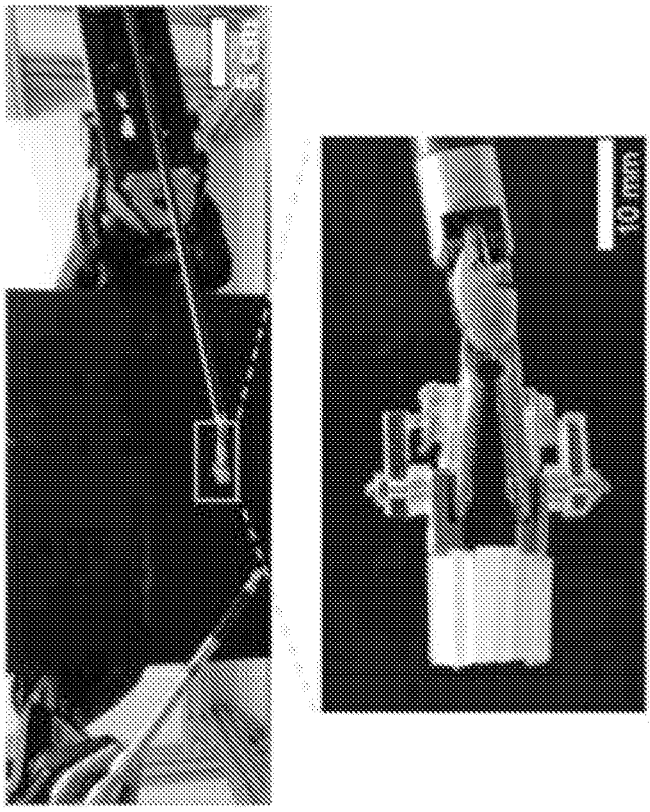


FIG. 14 B

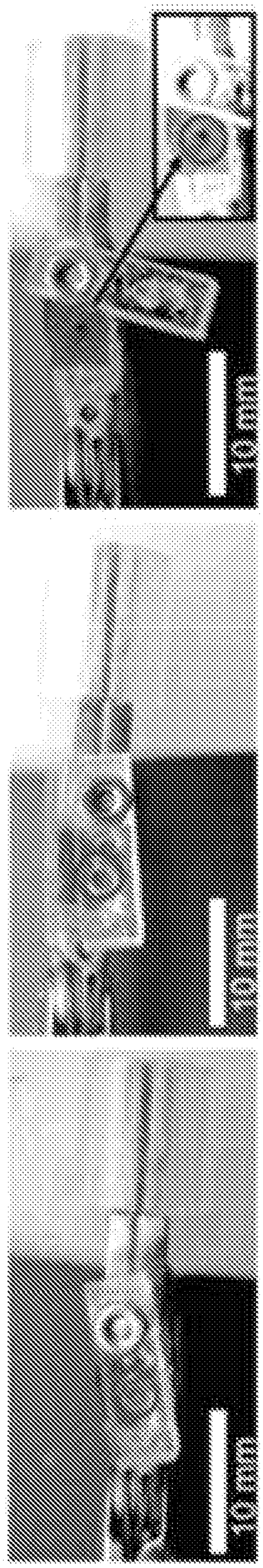


FIG. 14 C

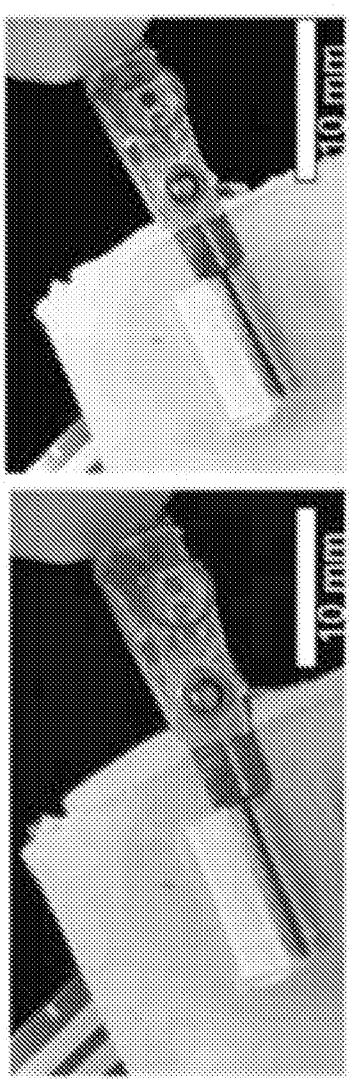


FIG. 14D

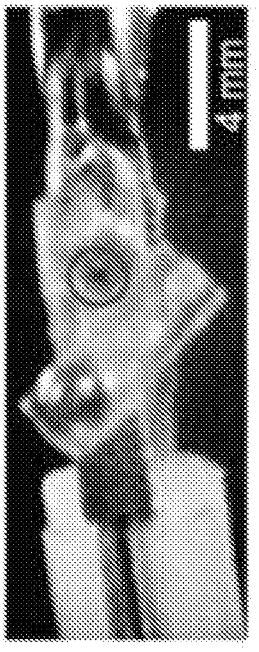


FIG. 14E

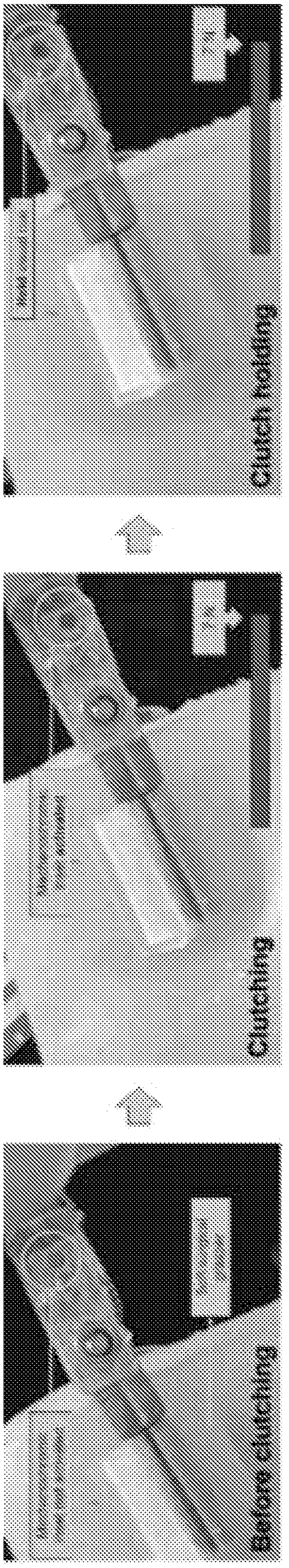


FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2016056908 A1
- US 20190151502 A1
- US 2003216732 A1
- US 2021041314 A1
- WO 2019155316 A1

### Non-patent literature cited in the description

- **PARK et al.** *Adv. Mater 2019,* 2019, vol. 31, 1808148
- **G. R. GOSSWEILER ; G. B. HEWAGE ; G. SORIANO ; Q. WANG ; G. W. WELSHOFER ; X. ZHAO ; S. L. CRAIG.** *ACS Macro Lett,* 2014, vol. 3, 216
- **G. R. GOSSWEILER ; T. B. KOUZNETSOVA ; S. L. CRAIG.** *J. Am. Chem. Soc.,* 2015, vol. 137, 6148